# EUROPEAN PATENT SPECIFICATION

(11) **EP 1 126 804 B1**
(45) Date of publication and mention of the grant of the patent: **09.08.2006**
(21) Application number: 99956940.3
(22) Date of filing: 04.11.1999
(51) Int. Cl.: A61F 9/007

(54) **KERATOME AND BLADE CHANGER**
KERATOM UND KLINGENWECHSLER
KERATOTOME ET CHANGEUR DE LAME

(30) Priority: 05.11.1998 US 107187 P
(43) Date of publication of application: 29.08.2001
(73) Proprietor: Insight Technologies Instruments, LLC, Milford, CT 06460 (US)
(72) Inventor: SUTTON, David, F., Milford, CT 06460 (US); LEVESQUE, Gaston, Meriden, CT 06450 (US)
(74) Representative: Ruffles, Graham Keith
(86) International application number: PCT/US1999/026159
(87) International publication number: WO 2000/025711

(56) References cited:
- EP-A- 0 442 156
- EP-A- 0 771 553
- US-A- 5 586 980
- US-A- 5 658 303

## Description

### FIELD OF THE INVENTION

The invention herein relates to a keratome having automated drive translation of a cutter head with a suspended stabilized reciprocating blade, an improved suction ring with entrance and cutting guideways for presenting and applying the cutter head and its suspended blade to the cornea, an applanator, drive means for automated translation of the cutter head, and a blade changer cooperating with the cutter head.

### BACKGROUND OF THE INVENTION

A keratome is an instrument used in ophthalmic surgery and, more particularly, in surgery to reshape the cornea for vision correction. A keratome incises a generally spherical segment of the cornea except for a connecting hinge. The cornea segment is lifted and held aside while the exposed truncated cornea surface is shaped for vision correction. Thereafter, the spherical cornea segment is repositioned to cover the shaped, truncated cornea surface. The cornea segment heals to the shaped surface, resulting in a reshaped cornea that acts as a corrective lens.

It will be appreciated that a keratome must achieve an accurately positioned, surgically precise cut with minimal tissue damage to enhance the healing process. Further, the cut must remain uncontaminated, also to aid the healing process and avoid irritation and infection.

It is desirable to measure the cornea and coordinate the extent of the incision with the size of cornea in order to remove a properly sized cornea segment and to provide an appropriate hinge in conjunction with the excised cornea segment.

In accurately positioning and performing a cornea cut, it is known to use a suction ring as an interface with the eye. Suction is used to temporarily secure the suction ring to the eye in a desired position.
Typically, a suction ring is secured to the sclera, near the periphery of and surrounding the cornea.

The suction ring may position and present a cutting instrument with respect to the cornea. Clearly, if any slippage or disengagement of the suction ring occurs, a correspondingly inaccurate cut may also occur. Suction rings occasionally do experience slippage or disengagement, and it is believed this occurs because of a poor interface with the surface of the sclera resulting in loss of suction or uneven suction along various segments of the ring. Currently, engagement of a cutting instrument with a suction ring is often difficult to achieve, because the engagement must be precise, making the engagement difficult to initiate, and because the cutting instrument can dislodge the suction ring.

Precision of the cornea cut requires proper positioning of a cutting instrument with respect to the cornea, which is achieved by the location and secure attachment of the suction ring, and also requires a very smooth operating cutting blade. Cutting instruments often use a reciprocating cutting blade to achieve a smooth, precise incision. Any flutter in the operation of the reciprocating cutting blade can cause a somewhat ragged incision, with consequent difficulties in replacing the cornea segment and smooth healing thereof.

For example, document EP-A-0 442 156 discloses a keratome comprising a fixation ring including an eye ring and a shoe defining a guideway for receiving a cutter head in mating sliding engagement and an entrance guideway for receiving the cutter head, the cutter head having a blade which is reciprocated by a drive unit for mounting and translationally moving the knife in the guideway.

The cornea cut must also remain uncontaminated, because any foreign matter in the incision may become encapsulated and cause irritation and possible infection. Cutting instrument designs which support a cutting blade on a bearing surface adjacent the area of the incision increase the risk of contamination. Contact between the cutting blade and the bearing surface creates friction and wear. This not only heats the cutting blade, but also sloughs off microscopic metal wear particles. These may lodge in the cornea incision, with undesirable effect.

There is also a need to quickly and easily load blades into the cutting instrument, and to remove blades after use.

Therefore, there is a need for a keratome with automated translation of a cutting instrument, including a suction ring that easily, accurately and securely positions a cutting instrument with respect to the cornea prior to and during cutting, and that provides a surgically precise, uncontaminated incision of a cornea segment.

### SUMMARY OF THE INVENTION

Accordingly, it is a principal object of the invention herein to provide a keratome for ophthalmic surgery.

It is an additional object of the invention to provide a keratome with automated translation of a cutting instrument across a suction ring.

It is a further object of the invention herein to provide a keratome that achieves accurate and secure engagement with the eye.

It is another object of the invention to provide a keratome that facilitates accurate, pre-cut measurement of the cornea.

It is also an object of the invention to provide a keratome including a cutting instrument that is easily and accurately engageable with a suction ring secured to the eye.

It is a further object of the invention to provide a keratome that achieves an accurate and smooth cut.

It is another object of the invention to provide a keratome that avoids contamination of the incision made on the cornea.

It is also an object of the invention to provide a keratome with simple and efficient changing of the blade in a cutting instrument.

The invention is defined in claim 1. Preferred embodiments of the invention are defined in the dependent claims.

According to certain aspects of the invention herein, the keratome generally comprises a cutting instrument mounted for automated translation in an automated drive unit, and having a cutter head guided into precision sliding engagement with the suction ring, the cutter head including a suspended, reciprocating blade. An applanator is receivable on the suction ring for measuring the segment of cornea to be cut.

Also according to further aspects of the invention, the keratome comprises a cutting instrument mounted for automated translation in an automated drive unit, and having a cutter head including a reciprocating blade with a cutting edge extending below a sole surface, and a suction ring to which the automated drive unit is removably secured. The suction ring includes an eye ring adapted to be secured to an eye by suction and defines a cornea aperture presenting the cornea of the eye for cutting a cornea segment. The suction ring further includes a shoe from which the eye ring extends, the shoe defining a cutting guideway configured for receiving the cutter head in precision mating sliding engagement for passing the cutting edge of the cutter blade over the cornea aperture and thereby incising the cornea segment. The shoe further defines an entrance guideway extending from and generally aligned with the cutting guideway, the entrance guideway configured for receiving the cutter head in orienting sliding engagement positively positioning the cutter head into the aforesaid precision mating sliding engagement in the cutting guideway. The automated drive unit is secured to the suction ring after being guided to position by the cutter head, and prior to incising the corner by translation of the cutting instrument and cutter head.

According to further aspects, the entrance guideway is partially defined by a guide hoop of the shoe, and the automated drive unit is secured to the guide hoop. The cutter head is cooperatively shaped to enter the guide hoop and progressively orient the cutter head for entry to the cutting guideway. The guide hoop is preferably positioned substantially at the transition between the entry guideway and the cutting guideway. Also, according to further aspects, the entrance guideway includes toe slots and toe ramps along the marginal edges of an entrance ramp portion of the shoe, for generally aligning the cutter head with the guide hoop, the shoe defines side rails extending from the guide hoop, and the cutter head has rail pockets slidingly receiving the side rails. Thus, there are multiple points of contact between the cutting instrument and the shoe defining the entrance guideway, serving to align the cutting instrument for sliding movement in the cutting guideway and to position the automated drive uni-t for attachment to the guide hoop prior to translating the cutter head. The foregoing configurations of the cutter head and suction ring provide easy engagement and accurate positioning of the cutting instrument and automated drive unit with respect to the suction ring.

Referring to another aspect, the suction ring includes a handle and the handle is preferably positioned opposite the entrance guideway of the suction ring, for manual stabilization of the suction ring and coordination of the suction ring and cutting instrument during introduction of the cutting instrument and automated drive unit.

According to still other aspects in the invention, a suction ring includes an eye ring defining a cornea aperture and a shoe, and the shoe defines a socket for receiving and positioning an applanator with a measuring surface in contact with a cornea of an eye presented through the cornea aperture, for measuring the size of the cornea to be incised. The suction ring is selected to adjust the extent of the incision, and the applanator measurement of the cornea assures there will be an appropriate hinge on the incised cornea segment.

According to a still further aspect of the invention, the cutting instrument engages against the suction ring at a desired extent of incision. A selection of suction rings are provided for selectively setting different extents of incision, which are then automatically achieved by the cooperation of the automated drive unit, cutting instrument and section rings.

According to additional aspects, a suction ring includes an eye ring with outer and inner contact surfaces shaped to engage the eye, and a suction channel defined between the inner and outer contact surfaces. The suction channel additionally defines a secondary distribution channel and a suction conduit opens to the suction channel and the secondary distribution channel. The secondary distribution channel ensures delivery and equalization of suction about the eye ring for secure attachment to the eye.

Also according to aspects of the invention herein, the cutting instrument includes a cutter head having a blade assembly. The blade assembly includes a metal cutting blade mounted to and extending from a blade holder to a cutting edge. The blade holder is preferably fabricated of a plastic, such as nylon. The blade holder defines a drive track transverse to the cutter blade and its cutting edge. The cutter head further defines a blade cavity generally accommodating and supporting the blade holder for reciprocal sliding movement. The cutter head defines a blade slot accommodating the cutting blade and extending to a blade opening from a foot of the cutter head, adjacent which the cutting edge is deployed for incising the cornea. The cutting blade is suspended with respect to and does not contact the cutter head.

According to additional aspects of the invention, the blade cavity defines one of a guide slot or guide bar, and the blade holder defines the other of the guide slot or guide bar, the guide bar extending into the guide slot in closely conforming mating sliding engagement to guide and stabilize the blade holder in its reciprocal movement within the cutter head. In a more particular aspect of the invention, the cutter head defines a substantially rectangular guide bar, and the blade holder defines a substantially rectangular guide slot accommodating the guide bar. The guide slot has an incrementally smaller width than the guide bar to preload the blade holder on the guide bar. The guide bar, and additional surfaces of the blade cavity, may be treated with a lubricous coating such as a nickel/Teflon® coating, to provide smooth reciprocation.

The cutter head further includes a blade shield and foot, the foot having projecting tongues for slidingly engaging the guide grooves of the shoe portion of the suction ring. The cutter head is characterized in that it does not contact the cutting blade, which is suspended extending from the blade holder. The cutting edge of the blade extends a selected distance below a forward or toe surface of the foot to establish a uniform depth of cut.

According to further aspects, the cutting instrument in the automated drive unit, to which the cutter head is secured, has a drive shaft terminating in an eccentric drive pin received in the drive track of the blade holder. A turbine is used to rotate the drive shaft at high rpm, which according to one aspect of the invention is between 8,000 and 16,000 rpm, and thereby reciprocate the blade holder and blade. The drive shaft is supported on bearings and located to isolate application of the drive force to the blade holder and through the drive pin, in cooperation with the cutter head.

The cutting instrument is reciprocally mounted in the automated drive unit. The automated drive unit is engageable with the suction ring, and the automated drive unit has drive means for advancing the cutting instrument across the suction ring and for retracting the cutting instrument after incising a cornea segment of an eye. The drive unit is removably secured to the suction ring and can be introduced in the suction ring after the suction ring is secured on the eye, guided by the cutter head. Thus, the suction ring is attached without the bulk of the cutting instrument, and automated drive unit, and the cutting instrument and automated drive unit are easily joined to the suction ring for cutting.

Also, according to aspects, the drive unit has a threaded shaft and worm gear and the drive unit includes a motor powering the threaded shaft. The motor may be an electric motor, and an electrical spike at the limit of travel may be used to reverse the direction of the cutting instrument for automated withdrawal of the cutting instrument. The cutting instrument may be inactivated during withdrawal.

According to other aspects, the cutting instrument includes a tubular body with a motor and a cutter head, and the cutter head mounts to the tubular body by means of a mounting shank and a bayonet shaped groove which positively orients the cutter head with respect to the tubular body. The bayonet style groove also provides for quick mounting and dismounting of the cutter head, in cooperation with an axial, self-aligning coupling. According to additional aspects of the invention, the motor of the cutting instrument is connected to a drive shaft for reciprocating a cutter blade in the cutter head, the connection being achieved by a coupling including a drive coupling member having ears received in mating slots of a positioning coupling member, and a positioning coupling member having pointed ears for guiding the drive ears into the mating slots.

According to further aspects of the invention, the keratome is provided with a blade insertion tool which includes, a shaft retractor for retracting the drive pin of a blade drive shaft from the cutter head blade cavity to provide clearance for inserting the blade, and an injector for inserting a cutter blade into the blade cavity and for displacing a used cutter blade out of the blade cavity.

Other objects, aspects and features of the invention will in part be understood by those skilled in the art and will in part appear from a perusal of the following description of the preferred embodiments and the claims, taken together with the drawings.

### DRAWINGS

FIG. 1 is a side elevation view of a keratome according to the invention herein, including a suction ring and a cutting instrument having a cutter head, and automated drive unit;
FIG. 2 is a bottom perspective view of a cutter head of the keratome of FIG. 1;
FIG. 3 is a perspective view of a blade holder for a cutting blade of the keratome of FIG. 1;
FIG. 4 is a perspective view of a blade for the keratome of FIG. 1;
FIG. 5 is a perspective view of a suction ring and applanator of the keratome according of FIG. 1;
FIG. 6 is a top view of the suction ring of FIG. 5;
FIG. 7 is a bottom view of the suction ring of FIG. 5;
FIG. 8 is an end view of the suction ring of FIG. 5;
FIG. 9 is a perspective view of the suction ring and applanator of FIG. 5;
FIG. 10 is a side elevation view, partially in section, of the suction ring and applanator of FIG. 5;
FIG. 11 is a perspective view of the keratome of FIG. 1, partially cut away;
FIG. 12 is an enlarged sectional view of the cutter head entering the suction ring of the keratome of FIG. 1;
FIG. 13 is an enlarged, partially sectional view of the cutting instrument engaged with the suction ring of the keratome of FIG. 1;
FIG. 14 is a perspective view of a portion of the keratome of FIG. 1, showing the cutting instrument engaged with the suction ring;
FIG. 15 is a perspective view a portion of the keratome of FIG. 1, similar to the view of FIG. 14 and additionally cut away;
FIG. 16 is side elevation view, partially cut away, of the cutting instrument component of the keratome of FIG. 1;
FIG. 17 is a sectional view of the keratome of FIG. 1, taken along the lines 17-17 of FIG. 11;
FIG. 18 is a foreshortened perspective view, partially cut away, of the keratome of FIG. 10;
FIG. 19 is a perspective view toward the bottom of the cutter head of the keratome of FIG. 1, illustrating its mounting configuration;
FIG. 20 is an enlarged view of the coupling between the cutter head drive shaft and the motor drive of the cutting instrument of the keratome of FIG. 1;
FIG. 21 is a perspective view of one of the positioning coupling element of FIG. 20;
FIG. 22 is a perspective view of the drive coupling element of FIG. 20;
FIG. 23 is a perspective view of the cutter head drive shaft of the keratome of FIG. 1;
FIG. 24 is a perspective view of the cutter head of the keratome of FIG. 1, and one element of a blade insertion tool;
FIG. 25 is a perspective view of the cutter head and the element of the blade insertion tool of FIG. 24 shown inserted in the cutter head, and a second element of the blade insertion tool inserting a cutter blade; and
FIG. 26 is a perspective view of the cutter head, and first and second elements of the blade insertion tools shown removing a cutter blade from the cutter head.

The same reference numbers refer to the same elements throughout the various figures.

### DESCRIPTION OF PREFERRED EMBODIMENTS

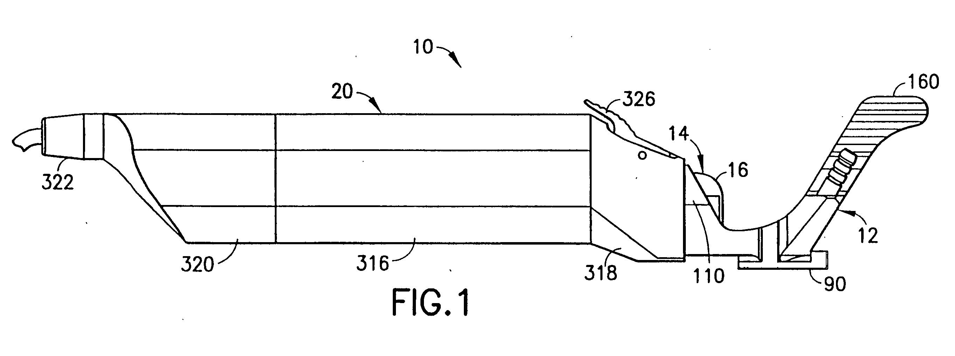

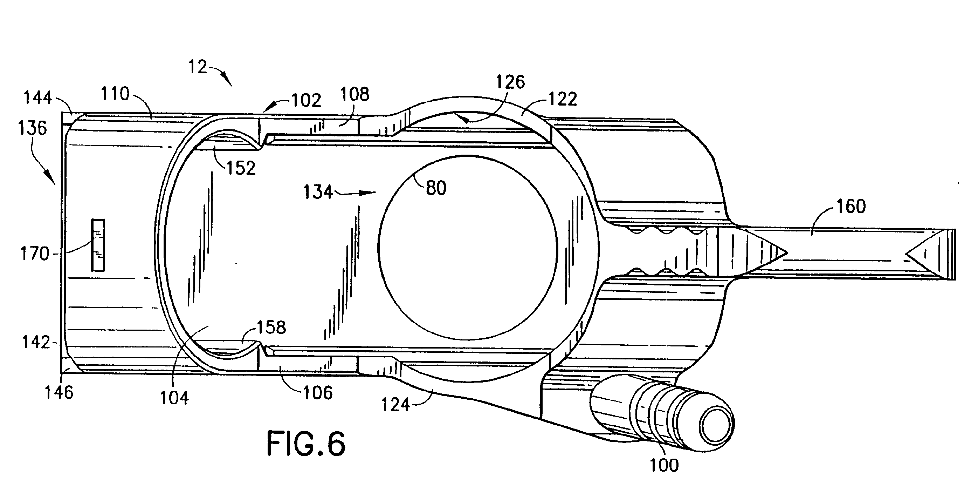

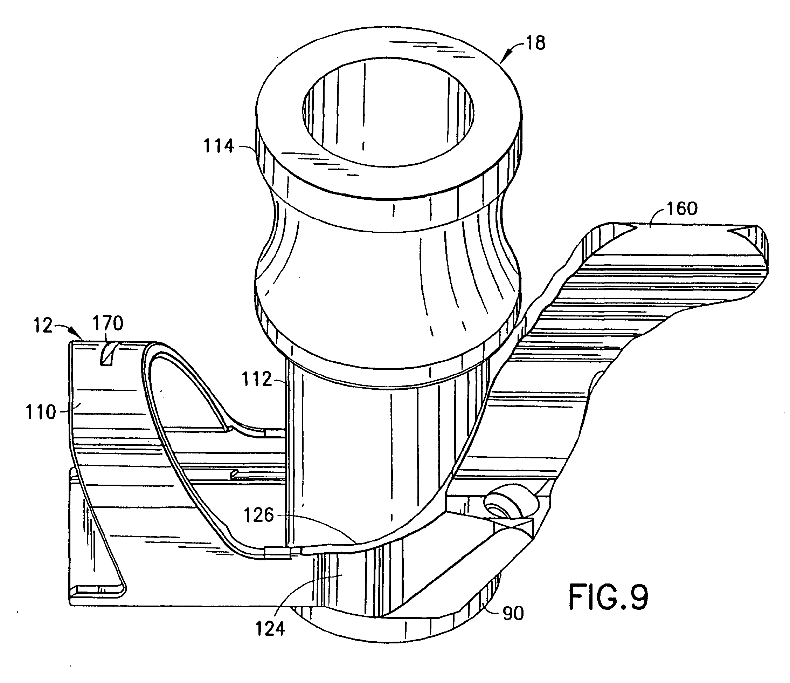

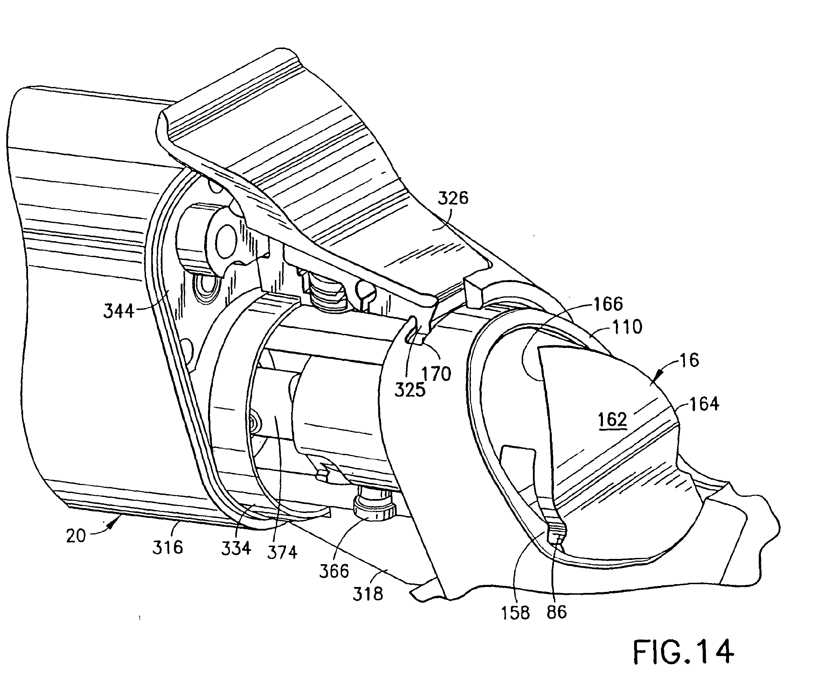

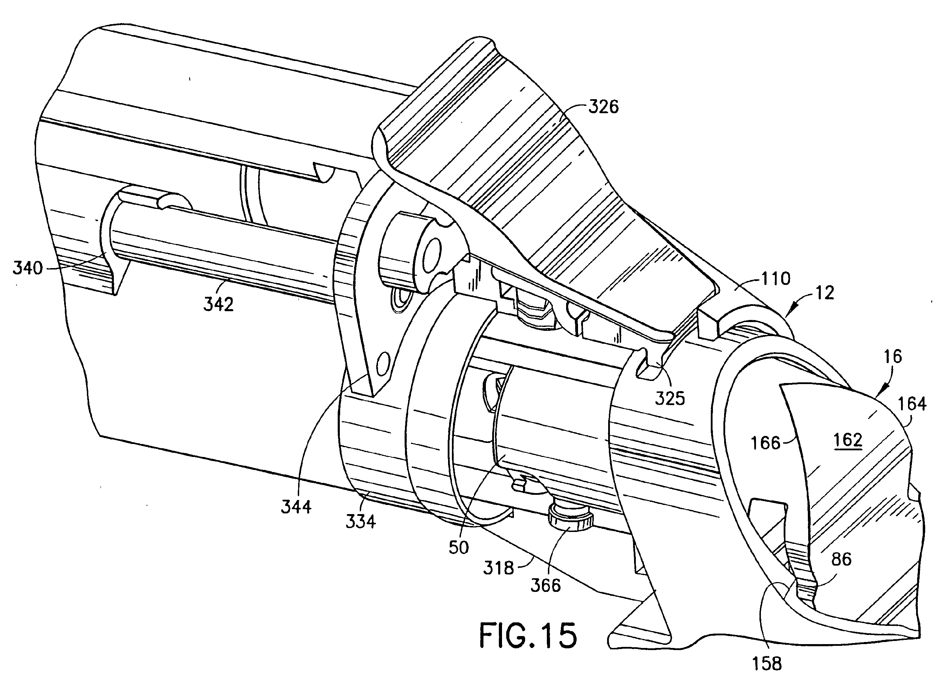

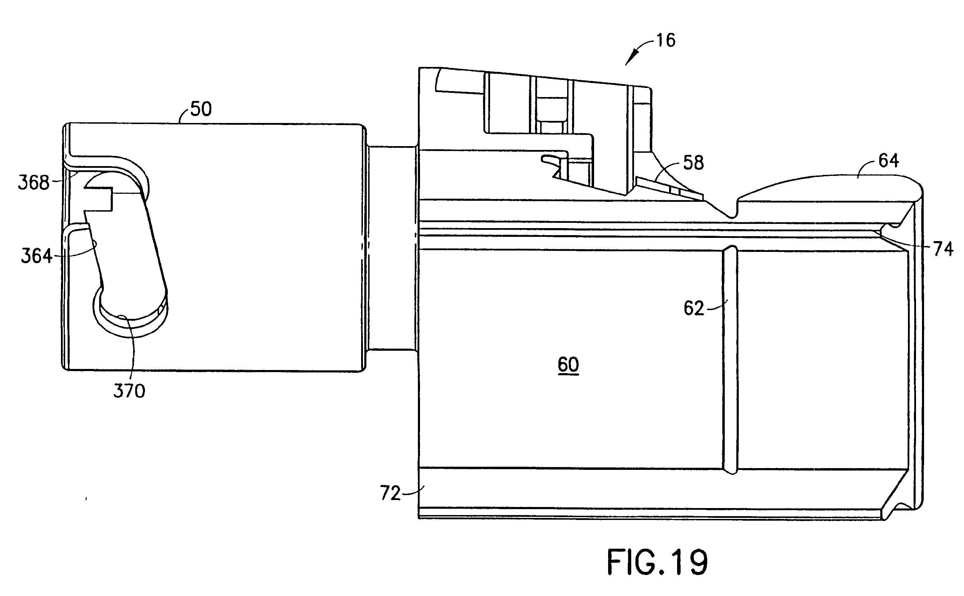

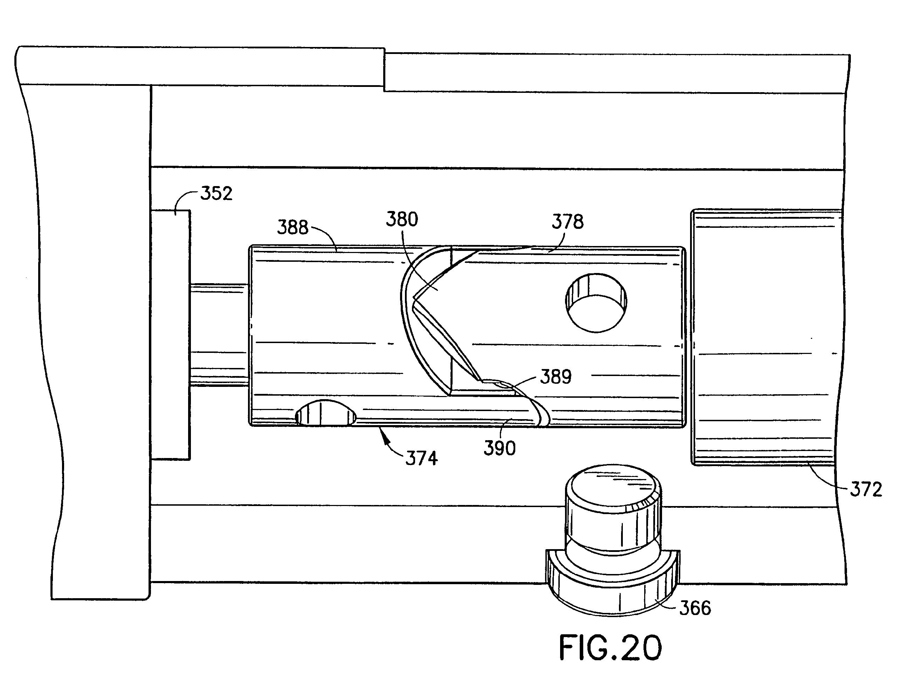

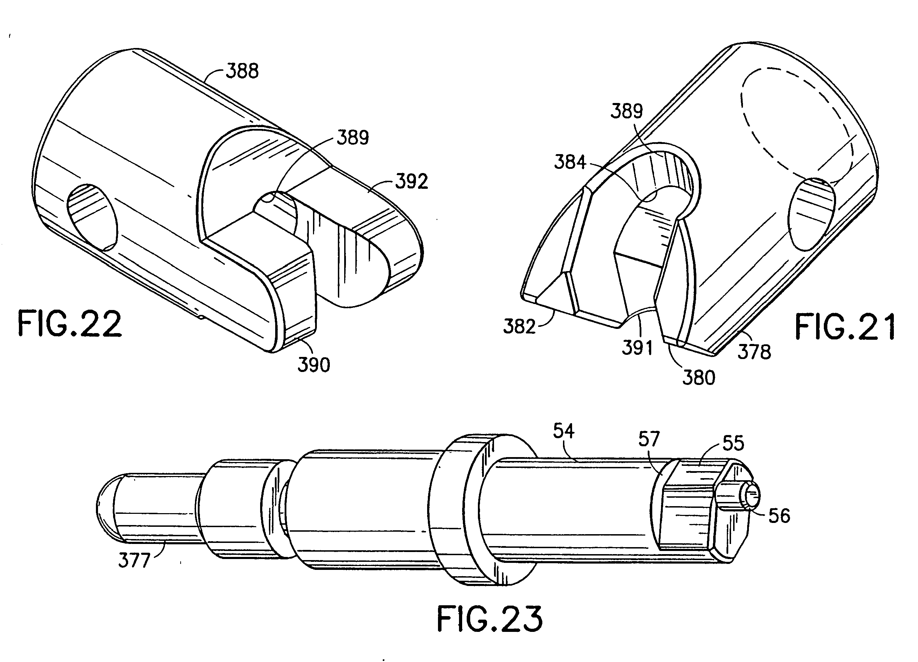

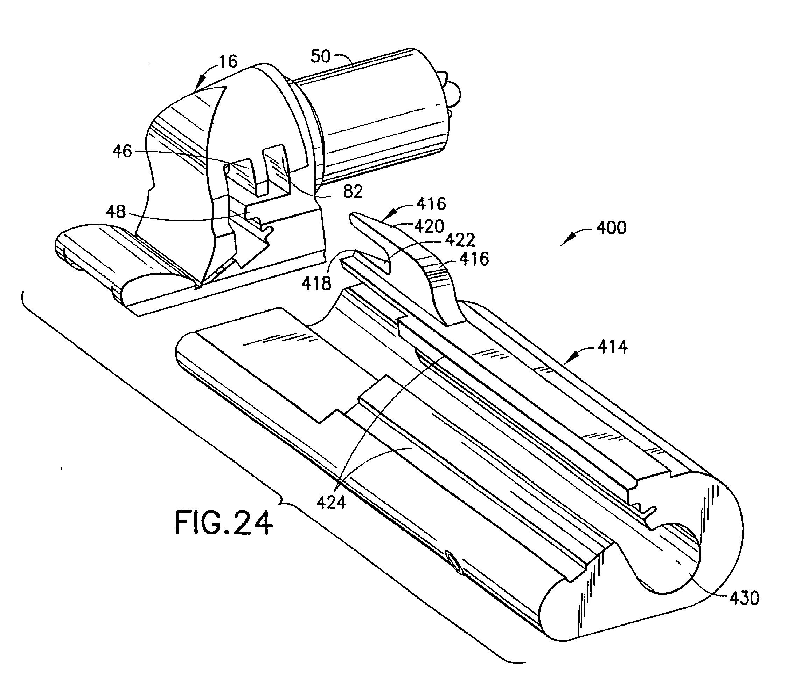

With references to FIGS. 1-23, there is illustrated a keratome 10 according to the invention herein that is used in ophthalmic surgery for removing a cornea segment. The keratome 10 generally comprises a suction ring 12 and a cutting instrument 14, having a cutter head 16 and an automated drive unit 20 for translating the cutter instrument 14 and its cutter head 16 across the suction ring 12. The keratome 10 also has a cooperating applanator 18, and a blade insertion tool 400 shown in FIGS. 24-26. Before discussing the keratome 10 and its operation, various component parts will be individually described.

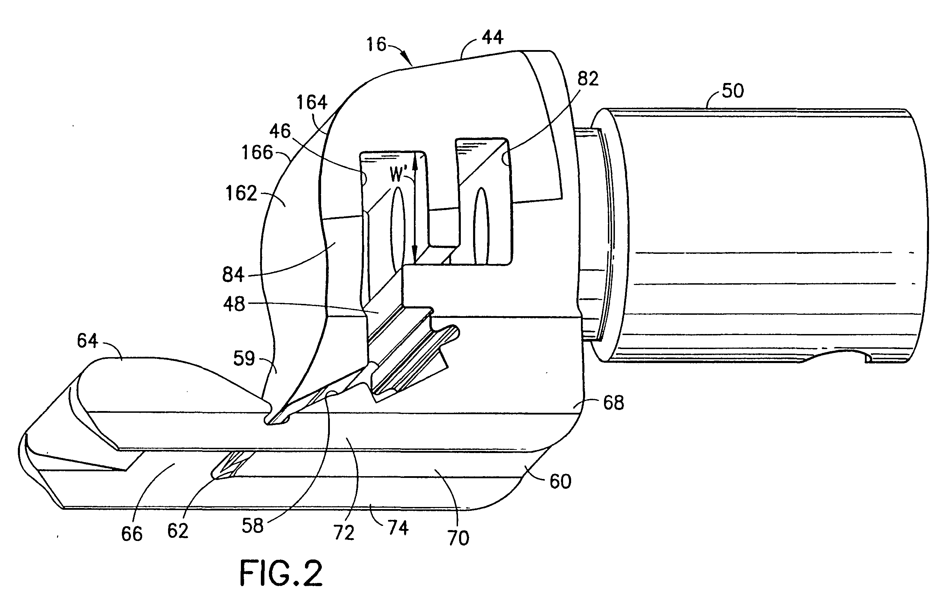

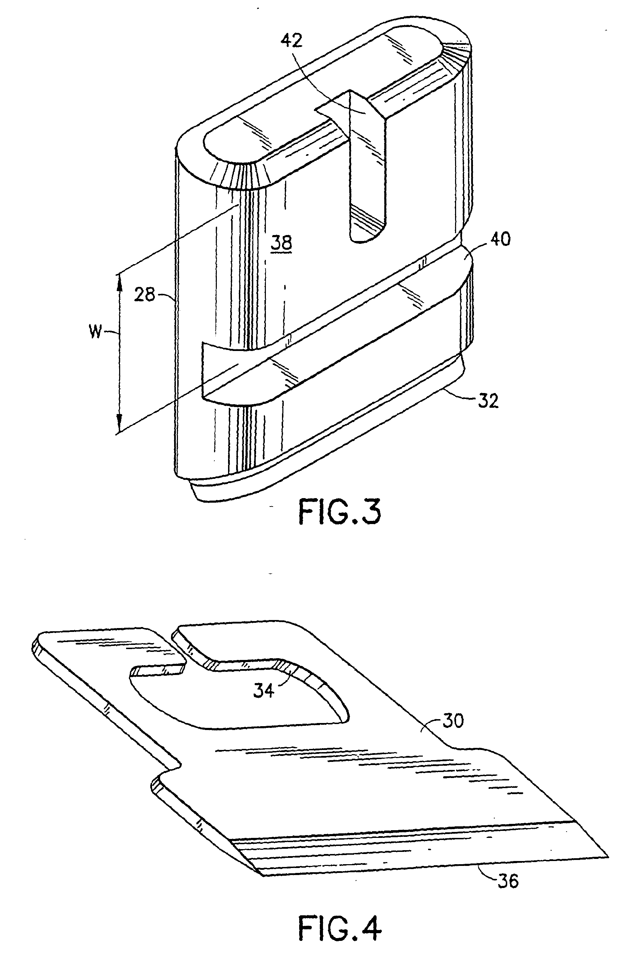

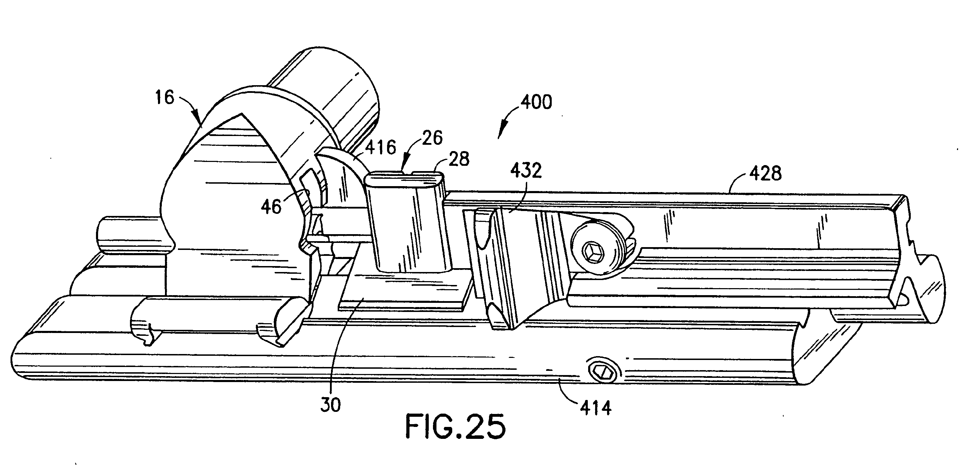

With reference to FIGS. 2-5, 11 and 25, the cutting instrument 14 of the keratome 10 includes the cutter head 16 and tubular drive 24. The cutter head 16 shown in FIG. 2, receives a blade assembly 26 seen in FIG. 25, with a blade holder 28 and a blade 30 of the blade assembly 26 being shown in FIGS. 3 and 4, respectively. The blade holder 28 is a shaped nylon body having a mounting stud 32 extending from the bottom thereof, and the blade 30 has an opening 34 which is received surrounding the mounting stud 32 to mount the blade on the blade holder, with the blade extending therefrom to a cutting edge 36. The blade holder 28 defines a guide slot 40 which extends across the back surface 38, parallel to the blade 30 and its cutting edge 36, and the back surface 38 further defines a drive track 42, perpendicular to the guide slot 40. The cutting edge 36 of the blade extends oppositely from the back surface 38 of the blade holder as seen for instance in FIG. 25.

The body 44 of the cutter head 16 is a one-piece, unitary structure defining a blade holder cavity 46 which closely embraces and guides the blade holder 28, but permits the blade 30 suspended from blade holder 28 to operate free from contact with the cutter head body 44. More particularly, the cutter head includes a guide bar 48 which is matingly received in the guide slot 40 in the back surface 38 of the blade holder 28, and the top of the blade holder and front surface of the blade holder also are slidingly engaged within the blade holder cavity 46. The blade holder 28 is fabricated of nylon has a width W from the guide slot to the top that is larger than the corresponding width W' from the guide bar 48 to the top of the blade holder cavity by approximately 0,012-0,024 mm (.0005-0010 inches). This provides a "preloaded" engagement of the blade holder on the guide bar and achieves a positive, supported, stabilized alignment of the blade holder on the guide bar during reciprocating movement. The surface of the blade cavity is preferably coated with a nickel/Teflon® material to provide smooth reciprocation, effectively lubricating the preloaded interface between the guide bar 48 and the guide slot 40.

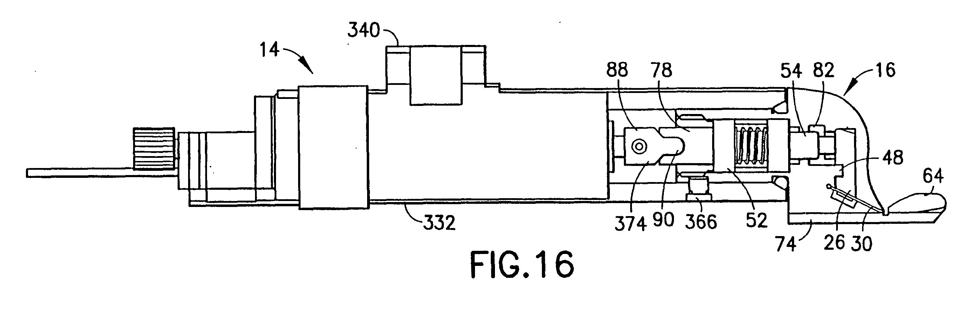

As best seen in FIGS. 2 and 16, the cutter head 16 further comprises a mounting shank 50 with bearings 52, which support a drive shaft 54 extending from the tubular drive 24 and having a drive pin 56 eccentrically located on the end thereof. The drive pin 56 extends into the drive track 42, so that when the drive shaft 54 is rotated, the drive pin 56 reciprocates the blade assembly 26. The drive shaft 54 is supported on bearings 52 which limit the extension of the drive shaft and drive pin into the blade holder cavity 46 in order to prevent the end of the drive shaft from contacting the blade holder and creating friction and binding, and also permits the drive shaft 54 to retract sufficiently to remove the blade assembly 26 from the blade holder cavity 46, as described below.

The body 44 of the cutter head 16 further defines a blade slot 58 extending from the blade holder cavity 46, in which the blade 30 is suspended by the blade holder without touching the body 44. A blade shield portion 59 of the body 44 is located above the suspended blade.

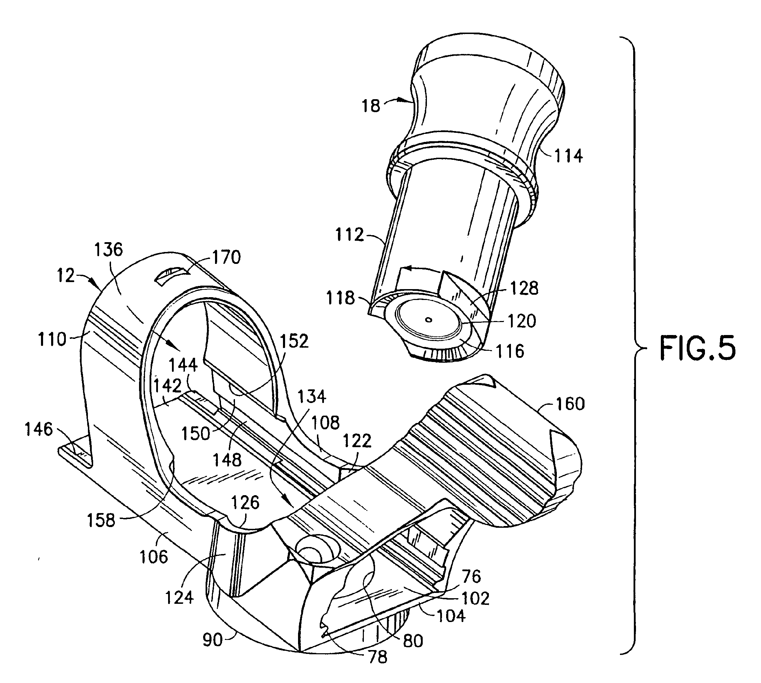

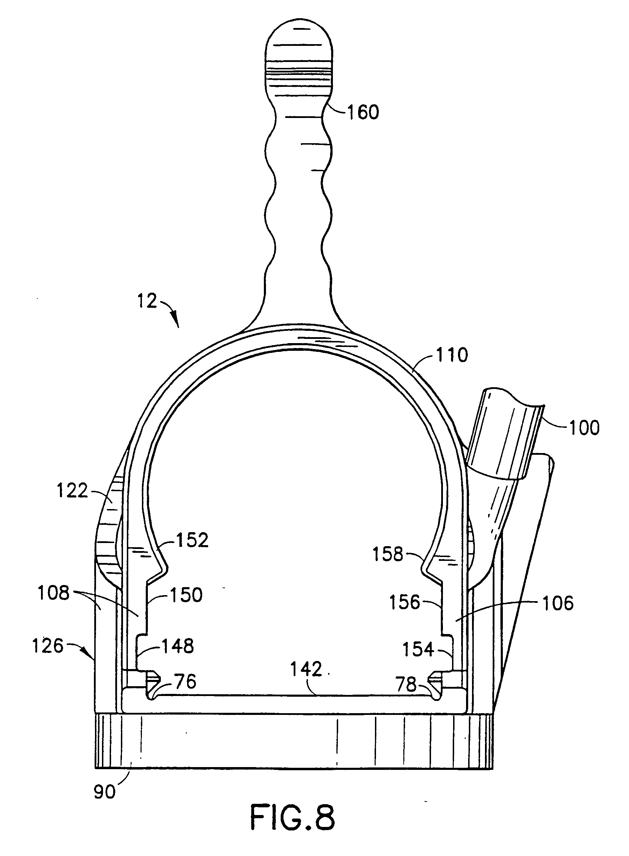

The body 44 further defines a foot 60 having a blade opening 62, through which the cutting edge 36 of the blade 30 protrudes. The foot 60 includes a toe portion 64 having a first sole surface 66 and heel portion 68 with a second sole surface 70, separated from the first sole surface 70 by blade opening 62. The blade opening 62 is formed where the blade slot 58 intersects the sole. The sole surfaces 66 and 70 are flanked by projecting tongues 72 and 74 which are respectively received in the guide grooves 76 and 78 of the suction ring 12 (FIGS. 5, 8 as discussed below). Thus, the cutter head 16 is engageable with the suction ring 12 to pass the cutting edge 36 of cutter blade 58 across the cornea when the suction ring is secured to an eye.

The first sole surface 66 of the cutter head 16 is stepped upwardly from the second sole surface 70, and the blade 30 extends through the blade opening 62 so that its cutting edge 36 is below sole surface 66. When the suction ring 12 is attached to an eye, the cornea extends upwardly through the cornea aperture 80 of cutting ring 12 and is pressed against the sole surfaces of the cutter head as the cutter head passes over the cornea aperture. The cornea is thereby cut to a depth determined by the extension of the cutting edge 36 of cutter blade 30 below sole surface 66. The lower, second sole surface 70 also supports the cornea, including the truncated surface thereof, and assists in presenting and stabilizing the cornea with respect to the blade edge 36 for cutting.

The cutting blade is disposed at an angle of approximately twenty-five degrees (25°) with respect to the sole surfaces of the cutter head, and it has been found that this angle produces a smooth and accurate cut. The angle, however, is not believed to be critical.

The cutting blade 30 does not touch the body 44 of the cutter head 16, but is positioned by the blade holder 28 in a suspended relationship to the surrounding structure. Therefore, there is no friction and wear generated between the cutting blade 58 and the cutter head body 54, and no debris is created which would contaminate the incision.

The cutter head 16 defines an additional blade tool cavity or access slot 82 spaced behind the blade holder cavity 46, and the drive shaft 54 extends therethrough to reciprocate the blade assembly 26. The blade holder cavity and the blade tool cavity may be joined, as illustrated, for ease of manufacture.

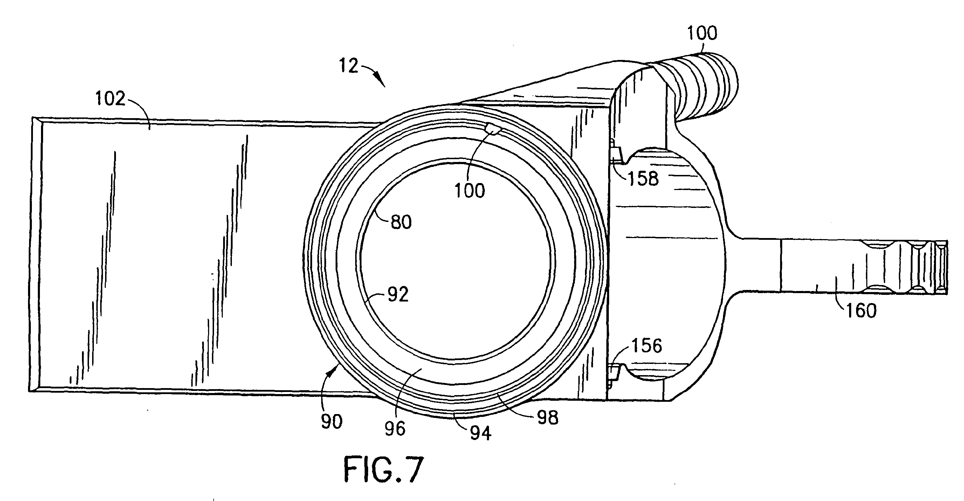

The suction ring 12 is shown in more detail in FIGS. 5-10. The suction ring 12 includes an eye ring 90. With particular reference to FIG. 7, the eye ring 90 has an inner annular contact surface 92 adjacent the cornea aperture 80, and an outer annular contact surface 94. A suction channel 96 is defined between the inner and outer contact surfaces 92, 94, and a secondary distribution channel 98 extends around the suction channel 96 and intersects with conduit stem opening 100. The suction ring is provided with a conduit stem 102 for connection with the source of negative pressure. The conduit stem is not shown in all of the figures for clarity of the remaining parts.

The suction ring 12 has a shoe 102 including a planer bottom wall 104 from which the eye ring 90 depends and which also defines the cornea aperture 80. The shoe 102 has side walls 106 and 108 upstanding from the bottom wall 104, and the side walls 106 and 108 merge with a guide hoop 110 at an entrance end of the suction ring 202 and with a handle 160 at the handle end of the suction ring 12.

The suction ring 12 cooperates with the applanator 18 in measuring the cornea prior to incising thereof. The applanator 18 has a generally tubular barrel portion 112 and a handle portion 114, which are integrally fabricated of an optically clear material, such as Lexan® . The applanator has a measuring surface 116 which is recessed below the end 118 of barrel 112. The depth of recess is preferably the same height the cutting edge 36 of the cutting blade 30 is disposed above the bottom wall of the cutter head 16 employed to incise the cornea. The measurement surface 116 is provided with a plurality of concentric rings 120 which are used in measuring the size of the cornea segment. In the embodiment shown, the inner ring is 8.5 millimeters in diameter, the central ring is 9.0 millimeters in diameter and the outside ring is 9.5 millimeters in diameter. The applanator is flat at 128 for clearance with respect to the suction ring.

The side walls 106 and 108 of the suction ring define opposed curved socket portions 122 and 124 of a socket 126, which positions the barrel 112 of the applanator 18 centrally over the cornea aperture 80. The flats 128 of the applanator accommodate portions of the side walls which define guideways for the cutter head 16, as more fully discussed below.

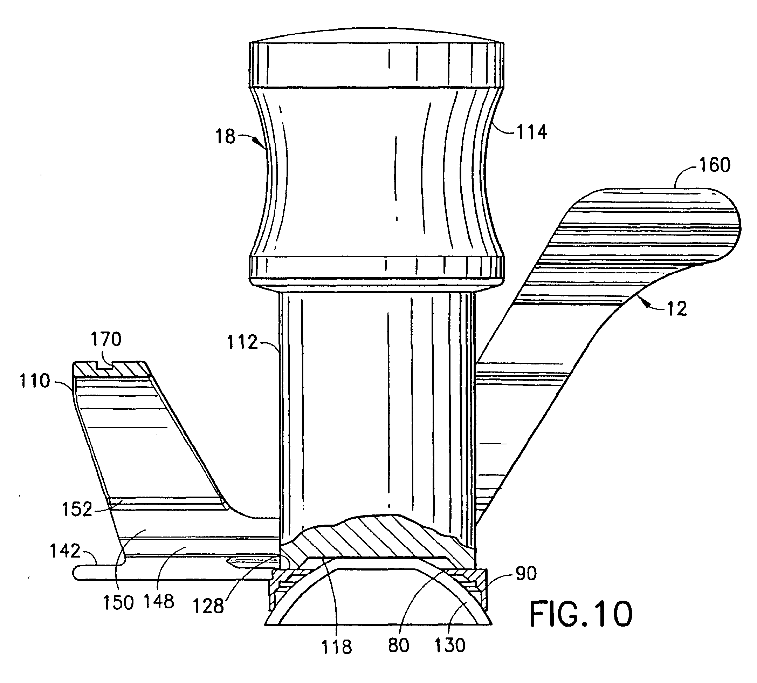

As best seen in FIG. 10, when the suction ring 12 is secured to eye, the cornea 130 protrudes through the cornea aperture 80 and extends above the bottom wall 104. When the applanator 18 is received in the socket 126 defined by the curved socket portions 122 and 124 of the suction ring side walls, and is also supported with its end 118 on the bottom wall 104, the cornea is in contact with the measuring surface 116 of the applanator. The surgeon views the concentric rings and cornea contact portion axially through the applanator 18. The diameter of the contact portion of the cornea is ascertained by reference to the concentric rings 120 on the measurement surface, and corresponds to the size of the cornea segment which will be incised by the cutting instrument 14. Upon ascertaining the size of the cornea segment, the applanator is removed from the socket 126.

As further discussed below, the suction ring is engaged by the cutting instrument to determine the extent of the incision, and several suction rings are available to provide the desired size. The applanator 18 is used to check the size before proceeding.

The keratome 10 is characterized by accurate engagement between the cutting instrument 14 and the suction ring 12, and by easy insertion of the cutting instrument into the suction ring. To this end, the shoe 102 of the suction ring defines a cutting guideway 134 configured for receiving the cutter head 16 in precision mating sliding engagement. Adjacent the precision cutting guideway 134 and extending to the entrance end of the suction ring is an entrance guideway 136, with the guide hoop 110 providing transition between the entrance guideway 136 and the cutting guideway 134.

The cutting guideway 134 includes precision guide grooves 76 and 78 respectively at the intersections of the bottom wall 104 and sidewalls 106, 108. The guide grooves 76, 78 respectively slidingly receive the tongues 72 and 74 of the cutter head 16. The precision guide grooves 76 and 78 are fully defined only in the cutting guideway 134 and the entrance guideway 136 serves to position the cutter head 16 for accurately and easily engaging the tongues 72 and 74 in the precision guide grooves 76 and 78.

The entrance guideway 136 begins with an entrance ramp portion 142 of the bottom wall 104, the entrance ramp portion 142 being provided with curbs 144 and 146 at its marginal edges. The lower portions of guide grooves 76, 78 are partially defined adjacent the curbs 144, 146, such that the tongues 72, 74 can drop into the partially defined guide grooves; however, this does not require precision alignment and is merely part of the function of the entrance guideway 136 in orienting and positioning the cutter head. At the entrance to the guide hoop 110, the side wall 236 defines a toe slot 148 and a toe slot ramp 150. The toe slot ramp 150 is positioned under a side rail 152 which extends inwardly from the toe slot ramp 150 and the interior of the guide hoop 110. The side wall 106 similarly defines toe slot 154, a toe slot ramp 156 and a side rail 158. The side rails 152 and 158 are discontinuous at the socket walls 124 and 126 as are the toe slot ramps 150 and 156.

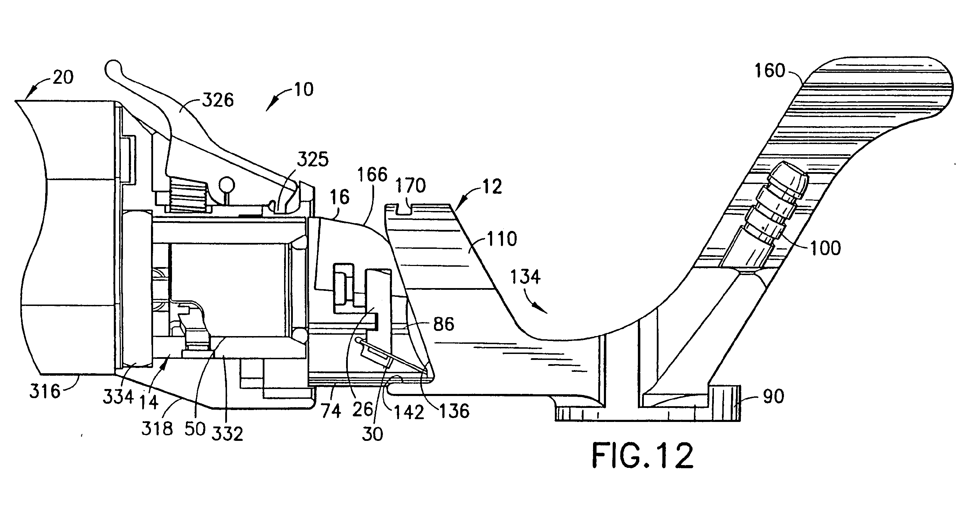

FIG. 12 illustrates entry of the cutting instrument 14 into the suction ring 12. Although the user's hands are not shown, it will be appreciated that the user will hold a handle 160 in one hand and will manipulate the automated drive unit mounting the cutting instrument 14 and cutter head with the other hand. It is advantageous to have both hands involved in the insertion of the cutter head 16 into the suction ring 12, in that the user can stabilize the suction ring 12 on the eye by sensing and compensating for any insertion forces, and also because the use of two hands tends to achieve better spacial orientation and physical coordination in making the insertion.

The toe 64 of the cutter head 16, including the leading portions of tongues 72 and 74, are placed against the entrance ramp portion 142 of the shoe 102. (The toe is just concealed by the guide hoop in FIG. 12.) If the tongues 72 and 74 are placed between the curbs 144 and 146, the toe is guided along bottom wall 104 into the guide hoop 110, and the tongues then enter the fully defined guide slots 76 and 78. If the cutting instrument 14 and automated drive unit 20 are not axially oriented with respect to the cutting guideway 134, a curved forehead 162 or one of flanking edges 164 and 166 defining the outer edges of the forehead 162 engages the guide hoop 110 and directs the cutter head 16 toward alignment. This engagement and continued forward movement of the cutter head 16, into entrance guideway 136 causes cutter head 16 to be axially aligned with the cutting guideway 134, and permits the tongues 72, 74 to enter the guide grooves 76 and 78.

If the toe 64 of cutter head 16 is somewhat tilted or initially misaligned, it will contact one of the toe slots 148, 154, or one of the toe slot ramps 150, 156, which will direct the toe into an adjacent respective toe slot. This provides an initial near alignment which is further corrected into axial alignment by contact of the forehead 162 or the transition edges 164 and 166 with the guide hoop 110, ultimately resulting in axially alignment of the cutter head 16 with the cutting guideway.

In all instances in which the toe 64 is inserted into the entrance guideway with the toe positioned under the side rails 152 and 158, the entrance guideway will accept the toe and by contact with the forehead 162 and transition edges 164, 166 the remainder of the cutter head will achieve alignment of the cutter head 16 for entry into the cutting guideway 134. It will be appreciated that the entrance guideway 136 tolerates misalignment on initial insertion and corrects the alignment as insertion into the entrance guideway 136 proceeds, making it extremely easy for the user to achieve the correct alignment of the cutter head 16 on the suction ring 12. The entrance guideway 136 has the additional benefit of providing entry to the suction ring 12 in an area spaced apart from the cornea aperture 80, thereby also preventing accidental damage to the cornea as a result of mis-engagement between the cutter head 16 and suction ring 12.

It will be noted that the cutter head 16 defines elongated side rail pockets 84 and 86 spaced upwardly from and parallel to the tongues 72, 74, and these pockets are matingly shaped with and slidingly the side rails 152, 158 of the suction ring 12.

With reference to FIGS. 1 and 11-17, the keratome 10 also comprises the automated drive unit 20, having a main housing 316, a front transition housing 318, and a rear transition housing 320 terminating in a cable cap 322 from which the wires necessary to power the keratome extend. The automated drive unit 20 of keratome 10 engages with the suction ring 12 and provides for automated reciprocal movement of the cutter head 16 across the cornea of an eye.

The front transition housing 318 is designed and configured to engage with guide hoop 110 and entrance ramp 142 of the suction ring 12 and, as seen in FIG. 5, the suction ring 12 defines a notch 170 receiving tongue 325 of a pivotal latch 326 of the front transition housing 318 to releasably secure the automated drive unit 20 and suction ring 12 together.

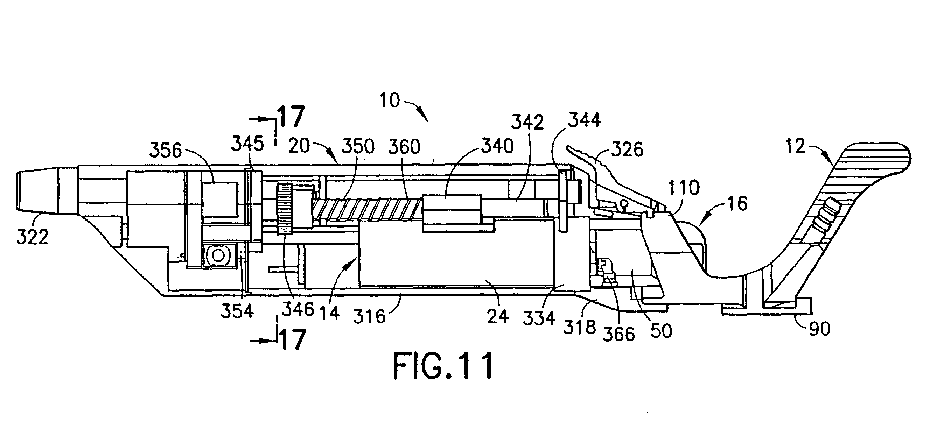

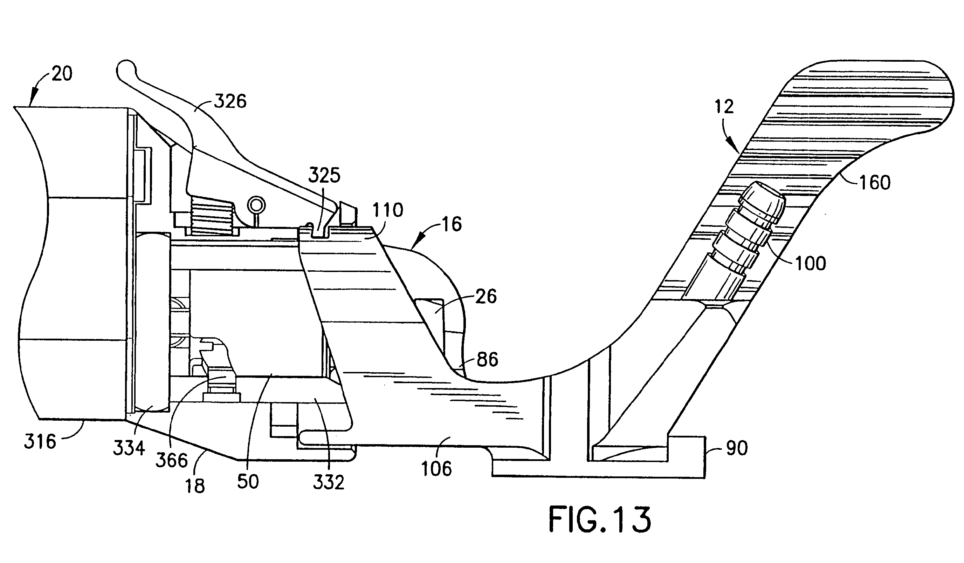

The cutting instrument 14 includes the cutter head 16. With reference to FIG. 16, the cutting instrument 14 defines a generally tubular body 332, and with reference to FIGS. 11-15, the tubular body is guided for reciprocal motion in the automated drive unit 310 by a bushing 334 at the front of the automated drive unit. The cutter head 16 is guided into the suction ring 12 in the manner discussed above. With-reference to Fig. 13, when the cutter head 16 has entered the guide hoop 110, the front transition housing 318 also receives and engages with the rear portion of the suction ring 12, and the tongue 325 of latch 326 is received in notch 170 to secure the automated drive unit 20 and cutting instrument 14 to the suction ring 12. The suction ring is, of course, secured to an eye in performing a cornea incision.

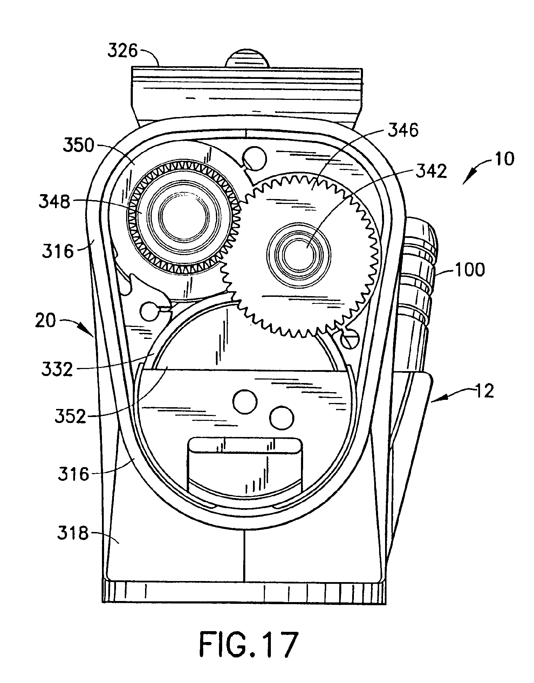

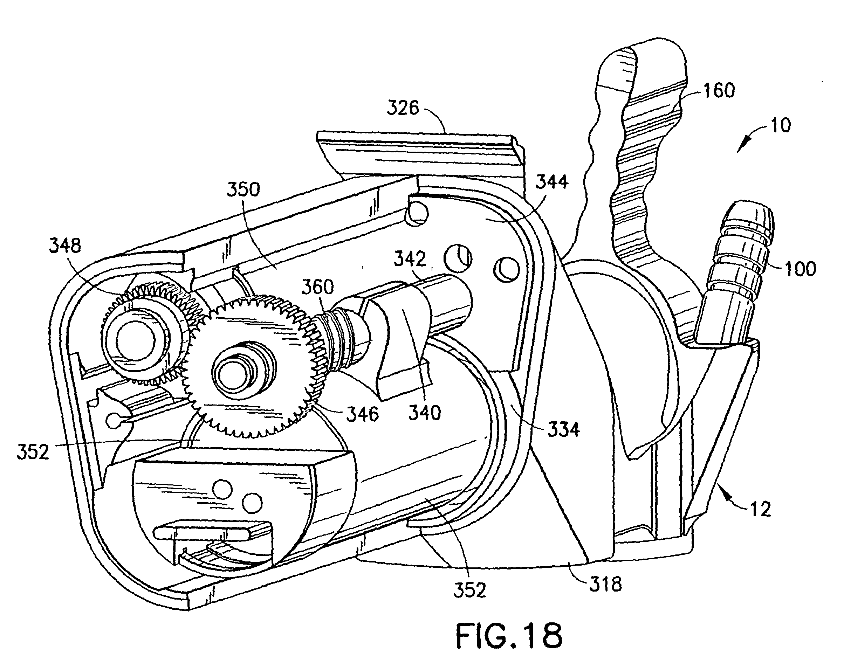

The automated drive unit 20 reciprocally translates the cutting instrument 14 on the suction ring 12. To this end, the cutting instrument 14 has a worm gear 340 extending from the tubular body 332 and the handle drive has a threaded shaft 342 (threads not shown) which is received in the worm gear 340. The threaded shaft 342 is rotatably mounted in a front plate 344 of the automated drive unit 20. The other end of shaft 342 is mounted to rear plate 345 and has a gear 346, which meshes with a gear 348 of a drive motor 350, as best seen in FIGS. 17 and 18. When the motor 350 spins gears 348 and 346, the threaded shaft 342 translates the cutting instrument 14 into the suction ring and across the eye opening thereof, to incise the cornea. When the worm gear 340 engages bushing 334, the load on the motor 350 is increased suddenly and dramatically, causing an electrical spike which is used to reverse the direction of the motor 350 to withdraw the cutting instrument 14. The electrical spike may also be used as a signal to stop operation of motor 352, which reciprocates the blade holder 28 and cutting blade 30 in the cutter head 16 of the cutting instrument. When the motor 350 moves the cutting instrument 14 back into the housing of the automated drive unit 20, a microswitch 354 is used to stop operation of the motor 350 when the cutting instrument 14 is fully retracted. An electronics package 356 controls these operations, with external input via operating switches.

A coil spring may be mounted surrounding shaft 342 to provide a bias load between the threaded shaft 342 and the worm gear 340, which prevents any gear lash which might otherwise cause an unsmooth motion of the cutting instrument 14 in the suction ring 12.

As the automated handle drive translates the cutting instrument, the cutter head enters the cutting guideway 134, the tongues 72 and 74 are received in the precision guide grooves 76, 78, and the side rails 152, 158 are received in the side rail pockets 84, 86. As the cutting instrument progresses within the cutting guideway 134, the tubular body 332 is received in the guide hoop 110. Thus, the cutting instrument 14 is fully supported and precisely positioned on the suction ring 12 as the cutting blade moves across the cornea aperture 80.

Before the cutting edge 36 of the cutting blade 30 moves completely across the cornea aperture, the cutter instrument 14 engages the guide hoop 110, limiting further insertion movement of the cutting instrument 14 and thereby preserving a hinge between an incised cornea section and the cornea. This engagement may alternatively be used to create the electrical spike for reversing the motor 350.

Thus, the keratome 10 is used by first engaging the suction ring 12 with the patient's eye and then using the applanator 18 to measure the proposed cornea section to be sure the proper suction ring has been selected for the desired cut. The cutter head 16 is inserted into the entrance guideway 136 of the suction ring 12. Engineered interference between the cutter head and the suction ring causes alignment with the cutting guideway 134 of the suction ring. The automated drive unit 20 locks onto the suction ring with the cutter head so aligned. The automated drive unit operates to translate the cutter head of the cutting instrument into the cutting guideway. The cutting guideway precisely positions the cutting blade as it incises the cornea. The cutting blade is reciprocated in making the cut, and the cutting blade is suspended with respect to contact with surrounding structure of the cutting instrument, and is stabilized for a smooth cut. At the far extent of the cut, the automated drive unit reverses to withdraw the cutter head, preferable deactivating the cutter head drive motor during the withdrawing translational movement. The automated drive unit provides smooth, steady, controlled movement of the cutter head.

With reference to FIGS. 11-16 and 19, the cutter head 16 has a cylindrical mounting shank 50 defining a bayonet mounting slot 364. The bayonet mounting slot cooperates with a pin 366 mounted to the tubular body 32 of the cutting instrument. More particularly, the bayonet mounting slot 364 defines an entry way 368, and a retention end 370 in which the pin 366 seats to lock the cutter head into the desired orientation on the tubular housing 332, and also provides for easy removal of the cutter head 16 for sterilization, or blade changes.

The cutting instrument 14 includes a coupling 374, best seen in FIGS. 16 and 20-23, connecting the motor 352 and a drive shaft 54 extending through bearings 52 into the cutter head 16 for oscillating a cutting blade, in the manner described above. The coupling 374 includes a first, positioning coupling member 378 having positioning ears 380 and 382, which are preferably pointed. The positioning coupling member 378 also includes a central opening 384 in which the end 377 of drive shaft 54 is received with the end 377 thereof protruding through the coupling member 378. The coupling 374 further comprises the drive coupling member 388, characterized by rounded ears 390 and 392 which fit snugly in slots 389 and 391 of the positioning coupling member 378. Drive coupling member 388 also defines an axial opening 389, which receives the end 377 of drive shaft 376 when the coupling members are assembled-together.

Therefore, when the cutter head 16 is removed from the remainder of the cutting instrument, the coupling members 378 and 388 separate, and when the cutter head is reattached, the drive ears 390 and 392 will either settle into the slots 389, 391 or will engage against the pointed ears 380, 382 and be directed into the slots 389, 391, for mating fit transmitting the rotation of the shaft of motor 352. As noted above, the drive shaft protrudes through the positioning coupling member 378 into opening 389 of the drive coupling member 388, for secure axial alignment of the coupling 374.

The keratome 300 is also provided with a blade insertion tool 400, shown in FIGS. 24-26, which operates with respect to the cutter head 16 after its removal from the cutting instrument 14 by disengagement of the bayonet mount. The function of the blade insertion tool is to retract the drive shaft 54 in order to remove the drive pin 56 of the drive shaft from the blade holder cavity 46 defined in the cutter head 16, in order that a cutting blade assembly 26 can be inserted without interference with the drive pin. To this end, the cutter head is provided with an blade tool cavity or access slot 82 behind the blade holder cavity 46, the end of the drive shaft partially cut away at 55 adjacent the drive pin and further defines a shoulder 57, and the drive shaft is biased to position the drive pin 56 in the blade holder cavity 46 and provide for retraction from the blade holder cavity 46.

With reference to FIG. 24, the blade insertion tool 400 comprises a shaft retractor and blade holder 414, including a tip 416 having ramped back surfaces 418 and 420 for engaging the shoulder 57 of the drive shaft and pushing the drive shaft rearwardly. The tip 416 also defines a slot 422 for receiving cutaway tip 55 of the drive shaft. The shaft retractor and blade holder 414 also defines a shaped blade support surface, generally referred to at 424, for receiving and positioning a cutting blade assembly 26 adjacent the cutter head 16, which is also supported on the shaft retractor and blade holder 414. FIG. 25 shows the shaft retractor with its tip 416 inserted in the cutter head 16, and the blade assembly 26 with its blade holder 28 adjacent the blade holder cavity 46 and its blade 30 adjacent blade slot 58.

With continued reference to FIG. 25, the blade insertion tool 400 further comprises an injector 428, which is received in groove 430 of the shaft retractor 414, and is slid therealong to push the blade assembly 26 supported on the blade support surface 424 into the cutter head 16. The injector 428 has a pivotally mounted stop member 432 which, when oriented toward the cutter head 16 as shown in FIG. 25, abuts and stops against the cutter head 16 when the blade holder 28 is positioned centrally in the blade holder cavity 46. Therefore, on withdrawal of the shaft retractor 414 and its tip 416, the drive pin 56 moves forward into the drive track 42 of the blade holder 28. It may be necessary to manually rotate the drive shaft 54 to seat the eccentric pin 56, but the drive track 42 is accurately positioned to receive it.

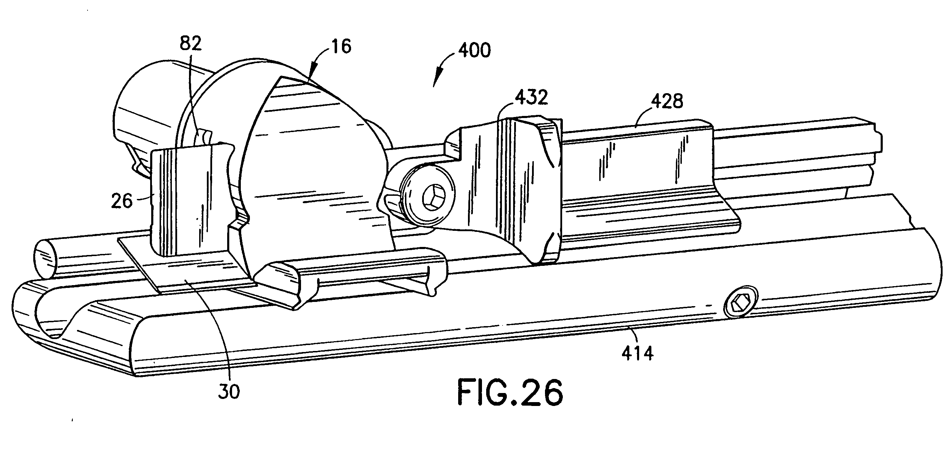

When the stop member 432 is pivoted upwardly, as shown in FIG. 26, the injector 428 may be slid into the blade holder cavity 46, displacing the blade assembly 26 therein and thus providing for removal of used blades.

It will be appreciated that in FIGS. 24-26, the cutter head 16 is shown disassociated from the other portions of the cutting instrument 14 and the automated drive unit 20 , so that the drive shaft may be retracted. The bayonet mount of the cutter head and the coupling 374 facilitate easy removal and replacement of the cutter head 16, and together with the blade insertion tool 400, make it a simple matter to change blade assemblies.

The keratome described above achieves very smooth incisions of the cornea in releasing a cornea segment, and does not damage or contaminate the cornea beyond the desired surgical incision. Accordingly, a keratome has been described which fulfill the objects of the invention herein. It will be appreciated by those skilled in the art that the keratome described above are illustrative of the invention, but that various changes and adaptations can be made without departing from the scope of the invention, which is limited only by the following claims and structures which may fairly fall therebetween.

## Claims

1. A keratome (10) having a cutting instrument (14) including a cutter head (15) having a reciprocating blade (30) with a cutting edge (36) extending below a sole surface (66, 70) of the cutter head, a suction ring (12) including an eye ring (90) adapted to be secured to an eye by suction and defining a cornea aperture (80) for presenting the outer layer of the cornea of the eye for cutting, a shoe (102) from which the eye ring extends, and
an automated drive unit (20) mounting the cutting instrument (14) and providng powered translational movement to at least the cutter head (16) thereof;
the shoe (102) of the suction ring (12) defining:
1) a cutting guideway (134) configured for receiving the cutter head (16) in precision mating sliding engagement when the cutting edge (36) of the blade (30) is positioned over the cornea aperture (80) of the eye ring (90), and
2) an entrance guideway (136) extending from and generally aligned with the cutting guideway (134), the entrance guideway (136) configured for receiving the cutter head (16) in orienting sliding engagement and positively positioning the cutter head (16) for precision mating sliding engagement with the cutting guideway (134);
said automated drive unit (20) and said suction ring (12) configured to be releasably attached to each other when said cutter head (16) is positioned in the entrance guideway (136) of the suction ring (12), and
said automated drive unit (20) operable to slidingly translate the cutter head (16) into the cutting guideway (134) and to withdraw the cutter head (16) therefrom,
wherein the cutter head (16) has a body (44) and a blade assembly (26),
1) the blade assembly (26) including a blade holder (28) having a drive track (42) and one of a guide slot (40) or a guide bar (48) substantially transverse to the drive track (42), the blade assembly (26) further including said blade (30) which is mounted to and extends outwardly from the blade holder (28) with said cutting edge (36) substantially parallel to the guide slot (40) or guide bar (48), and
2) the cutter head body (44) comprising
a) the sole surface (66, 70) for sliding engagement with a cornea, the sole surface (66, 70) having a blade opening (62),
b) a blade holder cavity (46) shaped to receive the blade holder (28) in reciprocating movement, the blade holder cavity (46) defining the other of the guide slot (40) or guide bar (48), wherein the guide bar (48) is received in the guide slot (40) for stabilizing the blade holder (28) in its reciprocating movement in the blade holder cavity (46), and
c) a blade slot (58) extending between the blade holder cavity (46) and the blade opening (62), wherein the blade (30) extends though the blade slot (58) and blade opening (62) with its cutting edge (36) positioned below the sole surface (66, 70) the blade slot (58) and blade opening (62) sized to accommodate the blade (30) without contact between the blade (30) and the cutter head body (44).

2. A keratome as defined in claim 1 wherein the guide bar (48) and guide slot (40) have substantially rectangular mating configurations.

3. A keratome as defined in claim 2 wherein the guide slot (40) has an incrementally smaller width than the guide bar (48) so that the guide bar (48) is tightly received in the guide slot (40).

4. A keratome as defined in claim 3 wherein the guide slot (40) is defined by the blade holder (28).

5. A keratome as defined in claim 1 wherein the guide slot (40) is defined by the blade holder (28).

6. A keratome as defined in claim 1 wherein the cutter head (16) is metal and the blade holder (28) is a plastic material.

7. A keratome as defined in claim 6 wherein the blade holder (28) is nylon.

8. a keratome as defined in claim 1 wherein the eye ring (90) has first and second generally concentric spaced apart surfaces (92, 94) sized and shaped for engaging the eye on or adjacent the cornea thereof, the eye ring (90) defining within the concentric spaced apart surfaces a cornea opening exposing a central portion of the cornea when the eye ring (90) is engaged with the eye, the eye ring (90) defining a suction channel (96) between the first and second concentric eye engaging surfaces (92, 94), and a secondary distribution channel (98) extending inwardly from the suction channel (96), and a stem (100) extending from the eye ring and having an opening for communicating suction with the eye ring (90), the stem (100) opening intersecting the suction channel (96) and secondary distribution channel (98).

9. A keratome as defined in claim 1 wherein the suction ring (12) has a guide hoop (110) upstanding from the shoe (102) thereof, the guide hoop (110) receiving and surrounding the cutter head (16) as it enters the entrance guideway (136).

10. A keratome as defined in claim 9 wherein the cutter head (16) and portion of the cutting instrument (14) adjacent thereto are surrounded and embraced by the guide hoop (110) as the automated drive unit (20) translates the cutter head (16) into the cutting guideway (134).

11. A keratome as defined in claim 9 wherein the guide hoop (110) provides the transition between the entrance guideway (136) and the cutting guideway (134).

12. A keratome as defined in claim 9 wherein the cutter head (16) has a toe (64) and the shoe (102) and guide hoop (110) of the suction ring (12) define flanking toe slots (148, 154) and toe slot ramps (150, 156) for guiding the toe (64) of the cutter head (16) into the entrance guideway (136).

13. A keratome as defined in claim 9 wherein the guide hoop and cutter head (16) are cooperatively configured to guide the cutter head (16) into the entrance guideway (136).

14. A keratome as defined in claim 13 wherein the cutter head (16) includes guide tongues (72, 74) depending from the edges of its sole surface (66, 70), and the suction ring (12) defines guide slots (76, 78) receiving the guide tongues (72, 74) as the automated drive unit (20) translates the cutter head (16) in the cutting guideway (134).

15. A keratome as defined in claim 13 wherein the automated drive unit (20) has a housing (316, 318, 320) surrounding the cutting instrument (14) and the housing (316, 318, 320) is matingly received and removably secured with the suction ring (12) when the cutter head (16) is in the cutting guideway (134).

16. A keratome as defined in claim 15 wherein the guide hoop (110) defines a notch (170) and the housing of the automated drive unit includes a latch (326) removably engaged in the guide hoop notch (170).

17. A keratome as defined in claim 9 wherein the automated drive unit (20) has a housing (316, 318, 320) surrounding the cutting instrument (14), and the housing (316, 318, 320) is matingly received and removably secured with the suction ring (12) when the cutter head (16) is in the cutting guideway (134).

18. A keratome as defined in claim 17 wherein the guide hoop (110) defines a notch (170) and the housing (316, 318, 320) of the automated drive unit (20) includes a latch (326) removably engaged in the guide hoop notch (170).

19. A keratome as defined in claim 1 wherein the shoe (102) from which the eye ring (90) extends has a bottom wall (104) surrounding the cornea aperture (80) and an applanator socket (126) extending upwardly from the bottom wall (104) surrounding the cornea aperture (80), and further comprising an applanator (18) removably received in the applanator socket (126), the applanator (18) having a sizing surface (116) which, when received in the socket (126), is positioned adjacent to and above the cornea aperture (80), wherein a cornea presented in the cornea aperture (80) extends to contact the applanator sizing surface (116), and the applanator sizing surface (116) includes indicia (120) indicating the size of the contact between with the cornea and the applanator sizing surface (116)

20. A keratome as defined in claim 1 wherein the automated drive unit (20) has a housing (316, 318, 320) surrounding the cutting instrument (14), and the housing (316, 318, 320) is matingly received and removably secured with the suction ring (12) when the cutter head (16) is in the cutting guideway (134).

21. A keratome as defined in claim 1 wherein the automated drive unit (20) has an elongated housing (316, 318, 320) and the cutting instrument (14) includes a generally tubular body (332) slidingly mounted in the elongated housing (316, 318, 320) for powered extension and retraction with respect thereto to translate the cutter head (16) in the cutting guideway (134).

22. A keratome as defined in claim 21 wherein the cutting instrument has a motor (3S2) that drives the reciprocating blade (30) in the cutter head (16) by a drive shaft (54), and the automatic drive unit (20) has an additional motor (350) that translates the cutting instrument (14) and cutting head (16).

23. A keratome as defined in claim 22 wherein the tubular body (322) of the cutting instrument (14) has a worm gear (340) mounted thereon, and the motor (350) of the automated drive unit (20) turns a threaded shaft (342) received in the worm gear (340) to translate the cutting instrument (14) and cutter head (16).

24. A keratome as defined in claim 23 wherein the motor (350) of the automated drive unit (20) is reversed at the desired extent of translation.

25. A keratome as defined in claim 24 wherein one of the cutting instrument (14) or cutter head (16) is mechanically stopped at the desired extent of translation, thereby causing an electrical spike in the motor (350) current used as a control signal to reverse the direction of translation.

26. A keratome as defined in claim 21 wherein the cutter head (16) is removably secured to the cutting instrument by a bayonet mount (364) of a cutter head mounting shank (50).

27. A keratome as defined in claim 26 wherein the drive shaft (54) of the cutter head and a drive shaft of the cutting instrument motor (352) are connected by an axially engageable coupling (374) that releases and engages as the cutter head (16) is removed and secured on the cutting instrument (14).

28. A keratome as defined in claim 27 wherein the coupling comprises a first drive coupling member (388) having two ears (390, 392) flanking a slot, and a second positioning coupling (378) having two slots (389, 391) receiving the two ears (390, 392) of the drive coupling member and two substantially pointed cars (380, 382) guiding the engagement of the coupling.

## Patentansprüche

1. Keratom (10) mit einem Schneidinstrument (14), das Folgendes einschließt: einen Schneidkopf (16), der eine hin- und hergehende Klinge (30) mit einer Schneide (36) hat, die sich unter einer Unterfläche (66, 70) des Schneidkopfs erstreckt, einen Saugring (12), der einen Augenring (90) einschließt, der so gestaltet ist, dass er durch Saugkraft an einem Auge befestigt werden kann und eine Hornhautöffnung (80) definiert, um die äußere Schicht der Hornhaut des Auges zum Schneiden anzulegen, ein Gleitstück (102), von dem sich der Augenring erstreckt, und
eine automatische Antriebseinheit (20), die auf dem Schneidinstrument (14) sitzt und wenigstens auf dessen Schneidkopf (16) eine kraftbetätigte Translationsbewegung überträgt;
wobei das Gleitstück (102) des Saugrings (12) Folgendes definiert:
1) eine Schneidführungsbahn (134) zum Aufnehmen des Schneidkopfs (16) in Präzisionspassungsgleiteingriff, wenn die Schneide (36) der Klinge (30) über der Hornhautöffnung (80) des Augenrings (90) positioniert ist, und
2) eine Eingangsführungsbahn (136), die sich von der Schneidführungsbahn (134) erstreckt und allgemein auf sie ausgerichtet ist, wobei die Eingangsführungsbahn (135) zum Aufnehmen des Schneidkopfes (16) in ausrichtendem Gleiteingriff und zum formschlüssigen Positionieren des Schneidkopfes (16) für einen Präzisionspassungsgleiteingriff mit der Schneidführungsbahn (134) konfiguriert ist;
wobei die genannte automatische Antriebseinheit (20) und der genannte Saugring (12) so konfiguriert sind, dass sie lösbar aneinander angebracht sind, wenn der genannte Schneidkopf (16) in der Eingangsführungsbahn (136) des Saugrings (12) positioniert ist, und
wobei die genannte automatische Antriebseinheit (20) die Aufgabe hat, den Schneidkopf (16) gleitend in die Schneidführungsbahn (134) zu verschieben und den Schneidkopf (16) aus ihr herauszuziehen;
wobei der Schneidkopf (16) einen Körper (44) und eine Klingenbaugruppe (26) hat,
1) wobei die Klingenbaugruppe (26) einen Klingenhalter (28) einschließt, der eine Antriebsbahn (42) und entweder einen Führungsschlitz (40) oder eine Führungsschiene (48) hat, der/die im Wesentlichen transversal zur Antriebsbahn (42) ist, wobei die Klingenbaugruppe (26) ferner die genannte Klinge (30) einschließt, die an dem Klingenhalter (28) angebracht ist und sich von ihm nach außen erstreckt, wobei die genannte Schneide (36) im Wesentlichen parallel zu dem Führungsschlitz (40) oder der Führungsschiene (48) ist, und
2) wobei der Schneidkopfkörper (44) Folgendes umfasst:
a) die Unterfläche (66, 70) für einen Gleiteingriff mit einer Hornhaut, wobei die Unterfläche (66, 70) eine Klingenöffnung (62) hat,
b) einen Klingenhalterhohlraum (46), der zum Aufnehmen des Klingenhalters (28) in einer Hin- und Herbewegung gestaltet ist, wobei der Klingenhalterhohlraum (46) entsprechend den Führungsschlitz (40) bzw. die Führungsschiene (48) definiert, wobei die Führungsschiene (48) in dem Führungsschlitz (40) aufgenommen ist zum Stabilisieren des Klingenhalters (28) in seiner Hin- und Herbewegung in dem Klingenhalterhohlraum (46), und
c) einen Klingenschlitz (58), der zwischen dem Klingenhalterhohlraum (46) und der Klingenöflhung (52) verläuft, wobei die Klinge (30) durch den Klingenschlitz (58) und die Klingenöffnung (62) verläuft, wobei ihre Schneide (36) unter der Unterfläche (66, 70) positioniert ist, wobei der Klingenschlitz (58) und die Klingenöffnung (62) größenmäßig zum Aufnehmen der Klinge (30) ohne Kontakt zwischen der Klinge (30) und dem Schneidkopfkörper (46) bemessen sind.

2. Keratom nach Anspruch 1, bei dem die Führungsschiene (48) und der Führungsschlitz (40) im Wesentlichen rechteckige zusammensteckkonfigurationen haben.

3. Keratom nach Anspruch 2, bei dem der Führungsschlitz (40) eine inkrementell kleinere Breite als die Führungsschiene (48) hat, so dass die Führungsschiene (48) eng anliegend in dem Führungsschlitz (40) aufgenommen wird.

4. Keratom nach Anspruch 3, bei dem der Führungsschlitz (40) von dem Klingenhalter (28) definiert wird.

5. Keratom nach Anspruch 1, bei dem der Führungsschlitz (40) von dem Klingenhalter (28) definiert wird.

6. Keratom nach Anspruch 1, bei dem der Schneidkopf (16) aus Metall und der Klingenhalter (28) aus einem Kunststoff ist.

7. Keratom nach Anspruch 6, bei dem der Klingenhalter (28) aus Nylon ist.

8. Keratom nach Anspruch 1, bei dem der Augenring (90) eine erste und eine zweite allgemein konzentrische Oberfläche (92, 94) hat, die voneinander beabstandet und für den Eingriff mit dem Auge an oder neben seiner Hornhaut bemessen und gestaltet sind, wobei der Augenring (90) in den konzentrischen beabstandeten Oberflächen eine Hornhautöffnung definiert, die einen zentralen Abschnitt der Hornhaut freilegt, wenn der Augenring (90) mit dem Auge in Eingriff ist, wobei der Augenring (90) einen Saugkanal (96) zwischen der ersten und der zweiten konzentrischen Augeneingriffsfläche (92, 94), und einen sekundären Verteilungskanal (98), der einwärts von dem Saugkanal (96) verläuft, und einen Schaft (100) definiert, der von dem Augenring verläuft und eine Öffnung für die Kommunikation von Saugkraft mit dem Augenring (90) hat, wobei die Öffnung des Schaftes (100) sich mit dem Saugkanal (96) und dem sekundären Verteilungskanal (98) schneidet.

9. Keratom nach Anspruch 1, bei dem der Saugring (12) einen Führungsbügel (110) hat, der aufrecht auf seinem Gleitstück (102) steht, wobei der Führungsbügel (110) den Schneidkopf (15) beim Eintritt in die Eingangsführungsbahn (136) aufnimmt und umgibt.

10. Keratom nach Anspruch 9, bei dem der Schneidkopf (16) und ein Abschnitt des Schneidinstruments (14) daneben von dem Führungsbügel (110) umgeben und umfasst werden, während die automatische Antriebseinheit (20) den Schneidkopf (16) in die Schneidführungsbahn (134) verschiebt.

11. Keratom nach Anspruch 9, bei dem der Führungsbügel (110) den Übergang zwischen der Eingangsführungsbahn (136) und der Schneidführungsbahn (134) bildet.

12. Keratom nach Anspruch 9, bei dem der Schneidkopf (16) eine Nase (64) hat und das Gleitstück (102) und der Führungsbügel (110) des Saugrings (32) flankierende Nasenausschlitze (148, 154) und Nasenschlitzrampen (150, 156) zum Führen der Nase (64) des Schneidkopfs (16) in die Eingangsführungsbahn (136) definiert.

13. Keratom nach Anspruch 9, bei dem der Führungsbügel und der Schneidkopf (16) kooperativ konfiguriert sind, um den Schneidkopf (16) in die Eingangsführungsbahn (136) zu führen.

14. Keratom nach Anspruch 13, bei dem der Schneidkopf (16) Führungszungen (72, 74) aufweist, die von den Rändern seiner Unterfläche (66, 70) herabhängen, und der Saugring (12) Führungsschlitze (76, 78) definiert, die die Führungszungen (72, 74) aufnehmen, während die automatische Antriebseinheit (20) den Schneidkopf (16) in der Schneidführungsbahn (134) verschiebt.

15. Keratom nach Anspruch 13, bei dem die automatische Antriebseinheit (20) ein das Schneidinstrument (14) umgebendes Gehäuse (316, 318, 320) hat und das Gehäuse (316, 318, 320) mit dem Saugring (12) zusammengesteckt und entfernbar befestigt ist, wenn der Schneidkopf (16) in der Schneidführungsbahn (134) ist.

16. Keratom nach Anspruch 15, bei dem der Führungsbügel (110) eine Einkerbung (170) definiert und das Gehäuse der automatischen Antriebseinheit eine Einschnappklinke (326) einschließt, die entfernbar in die Einkerbung (170) des Führungsbügels eingreift.

17. Keratom nach Anspruch 9, bei dem die automatische Antriebseinheit (20) ein das Schneidinstrument (14) umgebendes Gehäuse (316, 318, 320) hat und das Gehäuse (316, 318, 320) passend aufgenommen und entfernbar mit dem Saugring (12) befestigt ist, wenn der Schneidkopf (16) in der Schneidführungsbahn (134) ist.

18. Keratom nach Anspruch 17, bei dem der Führungsbügel (110) eine Einkerbung (170) definiert und das Gehäuse (316, 318, 320) der automatischen Antriebseinheit (20) eine Einschnappklinke (326) aufweist, die entfernbar in die Einkerbung (170) des Führungsbügels eingreift.

19. Keratom nach Anspruch 1, bei dem das Gleitstück (102), von dem sich der Augenring (90) erstreckt, eine die Hornhautöffnung (80) umgebende untere Wandung (104) und eine Applanatoraugenhöhle (126) hat, die sich von der die Hornhautöffnung (80) umgebenden unteren Wandung (104) nach oben erstreckt, und das ferner einen Applanator (18) umfasst, der entfernbar in der Applanatoraugenhöhle (126) aufgenommen ist, wobei der Applanator (18) eine Bemessungsfläche (116) hat, die, wenn er in der Augenhöhle (126) aufgenommen ist, neben und über der Hornhautöffnung (80) positioniert ist, wobei eine in der Hornhautöffnung (80) angelegte Hornhaut sich zum Berühren der Applanatorbemessungsfläche (116) erstreckt und die Applanatorbemessungsfläche (116) berührt und die Applanatorbemessungsfläche (116) Zeichen (120) aufweist, die die Größe des Kontakts zwischen der Hornhaut und der Applanatorbemessungsfläche (116) anzeigen.

20. Keratom nach Anspruch 1, bei dem die automatische Antriebseinheit (20) ein das Schneidinstrument (14) umgebendes Gehäuse (316, 318, 320) hat und das Gehäuse (316, 318, 320) passend aufgenommen und entfernbar mit dem Saugring (12) befestigt ist, wenn der Schneidkopf (16) in der Schneidführungsbahn (134) ist.

21. Keratom nach Anspruch 2, bei dem die automatische Antriebseinheit (20) ein längliches Gehäuse (316, 318, 320) hat und das Schneidinstrument (14) einen allgemein rohrförmigen Körper (332) einschließt, der zum kraftbetätigten Ausfahren und Einziehen in Bezug darauf zum Verschieben des Schneidkopfs (16) in der Schneidführungsbahn (134) gleitend in dem länglichen Gehäuse (316, 318, 320) sitzt.

22. Keratom nach Anspruch 21, bei dem das Schneidinstrument einen Motor (352) hat, der die hin- und hergehende Klinge (30) in dem Schneidkopf (16) durch eine Antriebswelle (54) antreibt, und die automatische Antriebseinheit (20) einen zusätzlichen Motor (350) hat, der das Schneidinstrument (14) und den Schneidkopf (16) verschiebt.

23. Keratom nach Anspruch 22, bei dem der rohrförmige Körper (322) des Schneidinstruments (14) ein daran angebrachtes Schneckengetriebe (340) hat und der Motor (350) der automatischen Antriebseinheit (20) einen in dem Schneckengetriebe (340) aufgenommenen Gewindeschaft (342) dreht, um das Schneidinstrument (14) und den Schneidkopf (16) zu verschieben.

24. Keratom nach Anspruch 23, bei dem der Motor (350) der automatischen Antriebseinheit (20) beim gewünschten Ausmaß der Translation umgekehrt wird.

25. Keratom nach Anspruch 24, bei dem entweder das Schneidinstrument (14) oder der Schneidkopf (16) mechanisch beim gewünschten Ausmaß der Translation angehalten wird, wodurch eine elektrische Spitze im Strom des Motors (350) verursacht wird, die als Steuersignal zum Umkehren der Translationsrichtung verwendet wird.

26. Keratom nach Anspruch 21, bei dem der Schneidkopf (16) durch eine Bajonetthalterung (364) eines Schneidkopfbefestigungsschaftes (50) entfernbar an dem Schneidinstrument befestigt ist.

27. Keratom nach Anspruch 26, bei dem die Antriebswelle (54) des Schneidkopfs und eine Antriebswelle des Schneidinstrumentmotors (352) durch eine axial einkuppelbare Kupplung (374) verbunden sind, die beim Abnehmen des Schneidkopfs (16) und Befestigen am Schneidinstrument (14) gelöst wird und in Eingriff kommt.

28. Keratom nach Anspruch 27, bei dem die Kupplung ein erstes Antriebskupplungselement (388) mit zwei einen Schlitz flankierenden Vorsprüngen (390, 392) und eine zweite Positionierungskupplung (378) mit zwei die zwei Vorsprünge (390, 392) des Kupplungsantriebselements aufnehmenden Schlitzen (389, 391) und zwei im Wesentlichen spitzen Vorsprüngen (380, 382) aufweist, die den Eingriff der Kupplung fuhren.

## Revendications

1. Kératome (10) comportant un instrument de coupe (14) engobant une tête de coupe (16) comportant une lame à déplacement alternatif (16) avec une arête de coupe (36) s'étendant au-dessous d'une surface de base (66, 70) de la tête de coupe, et une bague d'aspiration (12) englobant un anneau oculaire (90) adaptée à être fixé sur un oeil par aspiration et définissant une ouverture de la cornée(80) pour exposer la couche externe de la cornée de l'oeil en vue de la coupe, un patin (102) à partir duquel s'étend l'anneau oculaire, et
une unité d'entraînement automatique (20) sur laquelle est monté l'instrument de coupe (24) et permettant un déplacement par translation d'au moins la tête de coupe (16) correspondante ;
le patin de la bague d'aspiration (12) définissant :
1) une trajectoire de guidage de la coupe (134) configurée pour recevoir la tête de coupe (16) par engagement par glissement à ajustement précis lorsque l'arête de coupe (36) de la lame (30) est positionnée au-dessus de l'ouverture de la cornée (80) de l'anneau oculaire (90), et
2) une trajectoire de guidage d'entrée (136) s'étendant à partir de la trajectoire de guidage de coupe (134) et généralement alignée avec celle-ci, la trajectoire de guidage d'entrée (136) étant configurée pour recevoir la tête de coupe (16) par engagement par glissement à orientation et assurant le positionnement précis de la tête de coupe (16) en vue d'un engagement par glissement à ajustement précis dans la trajectoire de guidage de coupe (134) ;
ladite unité d'entraînement automatique (20) et ladite bague d'aspiration (12) étant configurées pour être fixées de manière amovible l'une à l'autre lorsque ladite tête de coupe (16) est positionnée dans la trajectoire de guidage d'rentrée (136) de la bague d'aspiration (12), et
ladite unité d'entraînement automatique (20) servant à déplacer par glissement translationel la tête de coupe (16) dans la trajectoire de guidage de coupe (134) et à retirer la tête de coupe (16) de celle-ci,
la tête de coupe (16) comportant un corps (44) et un assemblage de lame (26) ;
1) l'assemblage de lame (26) englobant un élément de retenue de la lame (28) comportant une piste d'entrainement (42) et un élément, une fente de guidage (40) ou une barre de guidage (48), pratiquement transversal à la piste d'entraînement (42), l'assemblage de lame (26) englobant en outre ladite lame (30) montée sur l'élément de retenue de la lame (28) et s'étendant vers l'extérieur de celui-ci, ladite arête de coupe (36) étant pratiquement parallèle à la fente de guidage (40) ou à la barre de guidage (48), et
2) le corps de la tête de coupe (44) comprenant :
a) la surface de base (56, 7), destinée à être engagée par glissement dans une cornée, la surface de base (66, 70) comportant ne ouverture de lame (62),
b) une cavité de l'élément de retenue de la lame (66) formée à recevoir l'élément de retenue de la lame (28) par déplacement alternatif, la cavité de l'élément de retenue de la lame (46) définissant l'autre élément, la fente de guidage (40) ou la barre de guidage (48), la barre de guidage (48) étant reçue dans la fente de guidage (40) pour stabiliser l'élément de retenue de la lame (28) lors de son déplacement alternatif dans la cavité de l'élément de retenue de la lame (46), et
c) une fente de la lame (58) s'étendant entre la cavité de l'élément de retenue de la lame (46) et l'ouverture de la lame (52), la lame (10) s'étendant à travers la fente de la lame (58) et l'ouverture de la lame (62), son arête de coupe (36) étant positionnée au-dessous de la surface de base (66, 70), la fente de la lame (58) et l'ouverture de la lame (62) étant dimensionnées de sorte à recevoir la lame (30) sans contact entre la lame (30) et le corps de la tête de coupe (44).

2. Kératome selon la revendication 1, dans lequel la barre de guidage (48) et la fente de guidage (40) ont des configurations complémentaires pratiquement rectangulaires.

3. Kératome selon la revendication 2, dans lequel la fente de guidage (40) a une largeur progressivement inférieure à celle de la barre de guidage (48), de sorte que la barre de guidage (48) est fermement reçue dans la fente de guidage (40).

4. Kératome selon la revendication 3, dans lequel la fente de guidage (40) est définie par l'élément de retenue de la lame (28).

5. Kératome selon la revendication 1, dans lequel la fente de guidage (40) est définie par l'élément de retenue de la lame (28).

6. Kératome selon la revendication 1, dans lequel la tête de coupe (16) est composée de métal, l'élément de retenue de la lame (28) étant composé d'un matériau plastique.

7. Kératome selon la revendication 6, dans lequel l'élément de retenue de la lame (28) est composé de nylon.

8. Kératome selon la revendication 1, dans lequel l'anneau oculaire (50) comporte des première et deuxième surfaces généralement concentriques espacées (92, 94) dimensionnées et formées de sorte à s'engager dans l'oeil ou sur la cornée qui y est adjacente, l'anneau oculaire (90) définissant dans les surfaces concentriques espacées une ouverture de la cornée exposant une partie centrale de la cornée lorsque l'anneau oculaire (90) est engagé dans l'oeil, l'anneau oculaire (90) définissant un canal d'aspiration (96) entre les première et deuxième surfaces concentriques d'engagement de l'oeil (92, 94), un canal de distribution secondaire (98) s'étendant vers l'intérieur à partir du canal d'aspiration (96), une tige (100) s'étendant à partir de l'anneau oculaire et comportant une ouverture en vue d'une communication par aspiration avec l'anneau oculaire (90), l'ouverture de la tige (100) coupant le canal d'aspiration (96) et le canal de distribution secondaire (98).

9. Kératome selon la revendication 1, dans lequel la bague d'aspiration (12) comporte un arceau de guidage (110) remontant du patin (102) correspondant, l'arceau de guidage (110) recevant et entourant la tête de coupe (16) lors de son entrée dans la trajectoire de guidage d'entrée (136).

10. Kératome selon la revendication 9, dans lequel la tête de coupe (16) et une partie de l'instrument de coupe (14) qui y est adjacente sont entourées et renfermées par l'arceau de guidage (110) lorsque l'unité d'entraînement automatique (20) entraîne le déplacement par translation de la tête de coupe (16) dans la trajectoire de guidage de coupe (134).

11. Kératome selon la revendication 9, dans lequel l'arceau de guidage (110) établit la transition entre la trajectoire de guidage d'entrée (136) et la trajectoire de guidage de coupe (134).

12. Kératome selon la revendication 9, dans lequel la tête de coupe (16) comporte une pointe (64), le patin (102) et l'arceau de guidage (110) de la bague d'aspiration (22) définissant des fentes de pointe flanquantes (148, 54) et des rampes des fentes de pointe (150, 156) pour guider la pointe (64) de la tête de coupe (16) dans la trajectoire de guidage d'entrée (136).

13. Kératome selon la revendication 9, dans lequel l'arceau de guidage et la tête de coupe (16) sont destinés à coopérer pour guider la tête de coupe (16) dans la trajectoire de guidage d'entrée (136),

14. Kératome selon la revendication 13, dans lequel la tête de coupe (16) englobe des languettes de guidage (72, 74), s'étendant à partir des bords de sa surface de base (66, 70), la bague d'aspiration (12) définissant des fentes de guidage (76, 78) recevant les languettes de guidage (72, 74) lorsque l'unité d'entraînement automatique (20) entraîne le déplacement par translation de la tête de coupe (16) dans la trajectoire de guidage de coupe (134).

15. Kératome selon la revendication 13, dans lequel l'unité d'entraînement automatique (20) comporte un boîtier (316, 318, 320) entourant l'instrument de coupe (14), le boîtier (316, 318, 320) étant reçu par ajustement par la bague d'aspiration (12) et fixé de manière amovible à celle-ci lorsque la tête de coupe (16) se trouve dans la trajectoire de guidage de coupe (134).

16. Kératome selon la revendication 15, dans lequel l'arceau de guidage (110) définit une encoche (170), le boîtier de l'unité d'entraînement automatique englobant un verrou (326) supporté de manière amovible dans l'encoche de l'arceau de guidage (170).

17. Kératome selon la revendication 9, dans lequel l'unité d'entraînement automatique (20) comporte un boîtier (316, 318, 320) entourant l'instrument de coupe (14), le boîtier (316, 318, 320) étant reçu par accouplement et fixé de manière amovible avec la bague d'aspiration (112) lorsque la tête de coupe (16) se trouve dans la trajectoire de guidage de coupe (134).

18. Kératome selon la revendication 17, dans lequel l'arceau de guidage (110) définit une encoche (170), le boîtier (316, 318, 320) de l'unité d'entraînement automatique (20) englobant un verrou (326) supporté de manière amovible dans l'encoche de l'arceau de guidage (170).

19. Kératome selon la revendication 1, dans lequel le patin (202) à partir duquel s'étend l'anneau oculaire (90) comporte une paroi inférieure (104) entourant l'ouverture de la cornée (80) et un orbite d'applanation (126) s'étendant vers le haut à partir de la paroi inférieure (104) entourant l'ouverture de la cornée (80), et comprenant en outre un élément applanateur (18) reçu de moyen amovible dans l'orbite d'applanation (126), l'élément applanateur (18) comportant une surface de dimension (116) qui, lors de sa réception dans l'orbite (126), est positionnée près de l'ouverture de la cornée (80) et au-dessus de celle-ci, la cornée exposée dans l'ouverture de la cornée (0) s'étendant de sorte à contacter la surface de dimension de l'élément applanateur (115), la surface de dimension d'applanation (116) englobant des repères (120) indiquant l'étendue du contact entre la cornée et la surface de dimension d'étalement (116).

20. Kératome selon la revendication 1, dans lequel l'unité d'entraînement automatique (20) comporte un boîtier (316, 318, 320) entourant l'instrument de coupe (14), le boîtier (316, 318, 320) étant reçu par ajustement et fixé de manière amovible avec la bague d'aspiration (12) lorsque la tête de coupe (16) se trouve dans la trajectoire de guidage de coupe (134),

21. Kératome selon la revendication 2, dans lequel l'unité d'entraînement automatique (20) comporte un boîtier allongé (316, 318, 30), l'instrument de coupe (14) englobant un corps généralement tubulaire (332) monté par glissement dans le boîtier allongé (316, 318, 320) en vue d'une extension et d'une rétraction entraînés par rapport à celui-ci, pour entraîner une translation de la tête de coupe (16) dans la trajectoire de guidage de coupe.

22. Kératome selon la revendication 21, dans lequel l'instrument de coupe comporte un moteur (352) entraînant la lame à déplacement alternatif (30) dans la tête de coupe (16) par un arbre d'entraînement (54), l'unité d'entraînement automatique (20) comportant un moteur additionnel (350) entraînant la translation de l'instrument de coupe (14) et de la tête de coupe (16).

23. Kératome selon la revendication 22, dans lequel le corps tubulaire (322) de l'instrument de coupe (14) comporte un engrenage à vis sans fin (340) qui y est montée, le moteur (350) de l'unité d'entraînement automatique (20) faisant tourner un arbre fileté (342) reçu dans l'engrenage à vis sans fin (340) pour entraîner la translation de l'instrument de coupe (14) et de la tête de coupe (16).

24. Kératome selon la revendication 23, dans lequel le moteur (350) de l'unité d'entraînement automatique (20) est inversé lors de l'extension voulue de la translation.

25. Kératome selon la revendication 24, dans lequel un des éléments, l'instrument de coupe (14) ou la tête de coupe (16), est arrêté mécaniquement lors de l'extension voulue de la translation, entraînant ainsi une pointe électrique dans le courant du moteur (350) servant de signal de commande pour inverser la direction de translation.

26. Kératome selon la revendication 21, dans lequel la tête de coupe (16) est fixée de manière amovible sur l'instrument de coupe par un support à baïonnette (364) de la tige de montage de la tête de coupe (50).

27. Kératome selon la revendication 26, dans lequel l'arbre d'entraînement (54) de la tête de coupe et un arbre d'entraînement du moteur de l'instrument de coupe (352) sont connectés par un accouplement à engagement axial (374) dégagé et engagé lors du retrait de la tête de coupe (16) et de sa fixation sur l'instrument de coupe (14).

28. Kératome selon la revendication 27, dans lequel l'accouplement comprend un premier élément d'accouplement d'entraînement (388) comportant deux pattes (390, 392) flanquant une fente, et un deuxième accouplement de positionnement (378) comportant deux fentes (389, 391) recevant les deux pattes (390, 392) de l'élément d'accouplement d'entraînement, et deux pattes pratiquement pointues (380, 382) guidant l'engagement de l'accouplement.
